(19) Europäisches Patentamt
European Patent Office
Office européen des brevets

(11) Publication number: 0 117 302
B1

(12) EUROPEAN PATENT SPECIFICATION

(45) Date of publication of patent specification: 27.06.90

(51) Int. Cl.$^5$: C 07 D 279/28, A 61 K 31/54

(21) Application number: 83112101.7

(22) Date of filing: 01.12.83

# (54) Phenothiazine derivatives.

(30) Priority: 02.12.82 GB 8234398
12.07.83 GB 8318832

(43) Date of publication of application:
05.09.84 Bulletin 84/36

(45) Publication of the grant of the patent:
27.06.90 Bulletin 90/26

(84) Designated Contracting States:
AT BE CH DE FR GB IT LI LU NL SE

(56) References cited:
GB-A- 805 886
GB-A- 808 239
GB-A- 969 251
US-A-2 905 668
US-A-2 956 996
US-A-3 112 310

The file contains technical information submitted after the application was filed and not included in this specification

(73) Proprietor: THE WELLCOME FOUNDATION LIMITED
160 Euston Road
London NW1 2BP (GB)

(72) Inventor: Coker, Geoffrey George
80 Pickhurst Park
Bromley Kent (GB)
Inventor: Findlay, John William Addison
Rt.2 Box 514 Cascade Drive
Chapel Hill North Carolina (US)
Inventor: Leighton, Harry Jefferson
4500 Cheshire Court
Durham North Carolina (US)

(74) Representative: Schwabe, Hans-Georg, Dipl.-Ing. et al
Patentanwälte Schwabe, Sandmair, Marx
Stuntzstrasse 16
D-8000 München 80 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 117 302 B1

Courier Press, Leamington Spa, England.

(56) References cited:
Arzneimittelforschung 19(8), 1193-8 (1969)
Fortschritte der Arzneimittelforschung 5, 292-3 (1963)

CHEMICAL ABSTRACTS, vol. 76, no. 1, 03.01.72, Columbus, OH (US); A.S.GRITSENKO et al.: "Synthesis of phenothiazines. XXXI. 10-v-alkylaminopropionyl and 10-b-alkylaminopropionyl derivatives of substituted phenothiazines", p. 335, no. 3774k

CHEMICAL ABSTRACTS, vol.87, no.13, 26 Sept. 1977, p.61, abstract 95744d; Columbus Ohio (US) R.NACHEVA et al.: "Study of the relation between biological activity and quantum-chemical parameters of some promazime derivatives".

**Description**

The present invention relates to new phenothiazine derivatives exhibiting anti-histamine and antiallergic activity, to a process for preparing them, to pharmaceutical compositions containing them and to their use in medicine.

US Patent No. 2 530 451 discloses a group of 9-(dialkylaminoalkyl)phenothiazines with antihistamine activity, the most outstanding of which is the compound named and hereinafter referred to by its generic name, promethazine (10-(2-dimethylaminopropyl)phenothiazine). Promethazine has gained a fair degree of clinical acceptance as a transquilizer and as an antihistamine.

GB—PS 808 239 discloses certain phenothiazine derivatives, in particular various 10-aminoalkyl-phenothiazines having an ester group in the 3-position of the phenothiazine nucleus. The compounds are said to be *inter alia* potentiators of narcosis.

In C.A.76 (1972) 377ka process for preparing phenothiazines substituted in the 10-position is disclosed. No indication to the use of said compounds is given.

The US—A—2 956 996 discloses alkylated phenothiazinecarboxamide derivatives which have utility as general central nervous system depressants.

US—A—3 112 310 discloses 10-alkylaminoalkylphenothiazine-2-carboxamides. It is mentioned that these compounds are useful because they show activity as depressants of the central nervous system and possess antihypertensive activity.

In US—A—2 905 668, phenothiazine derivatives have a piperazine substituent in the 10-position of the phenothiazine nucleus are disclosed. The compounds are very active as sedatives, antiemetics and cataleptics.

The antihistamines now in use, including diphenylhydramine, the pheniramines, pyrilamine, promethazine and triprolidine have one potential disadvantage in common; they all cause sedation or drowsiness in some patients. Promethazine also has the additional disadvantage that it has potent anticholinergic activity.

A novel group of compounds having antiallergic activity *in-vivo* as defined by blockade of anaphylactoid activity has now been discovered. Some of these compounds also have good antihistamine activity and appear to be substantially free of CNS side-effects and to possess markedly less anticholinergic activity than promethazine.

Accordingly this invention provides a compound of the formula (I)

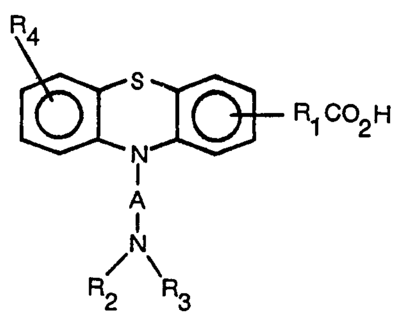

(I)

or a salt thereof; wherein

$R_1$ is a $C_{1-7}$ bivalent aliphatic hydrocarbon group or a single bond;

$R_2$ and $R_3$ are the same or different and are each hydrogen,. $C_{1-4}$ alkyl or taken together with the nitrogen comprise a pyrrolidine, piperidine or morpholine ring;

$R_4$ which is attached at the 7- or 8-position is hydrogen, halogen, $C_{1-4}$ alkoxy, $C_{1-4}$ alkyl optionally substituted by one to three halogen atoms; or a group $R_1$ $CO_2H$ as hereinbefore defined; and

A is $C_{1-4}$ alkylene;

other than the compound of formula (1) wherein $R_1CO_2H$ is $-CH_2-CO_2H$ attached at the 2-position of the phenothiazine ring, $R_4$ is hydrogen and $-ANR_2R_3$ is diethylamino ethyl.

Of the compounds of formula (I) those of formula (II) are preferred.

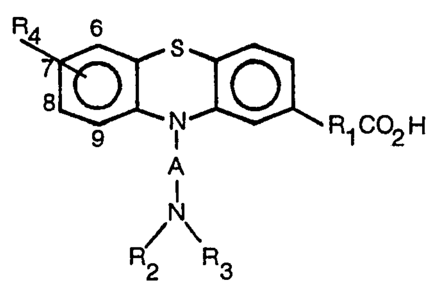

(II)

or a salt thereof; wherein $R_1$ to $R_4$ and A are as defined in relation to the formula (I).

Suitably $R_1$ is a straight or branched, saturated or unsaturated $C_{1-7}$ bivalent aliphatic hydrocarbon group or a single bond. Suitably $R_1$ is a $C_{1-3}$ hydrocarbon group or a single bond. Suitably $R_1$ contains at the most one double bond. Preferably $R_1$ is a group $—(CH_2)_n—$ wherein n is an integer 0 to 3, or a group $—CH{=}CH—$ or a group $—CH(CH_3)(CH_2)_m—$ where m is 0 to 1, or a group $—C(CH_3)_2—$. Most preferably $—R_1CO_2H$ is a group $—CH_2CO_2H$.

Suitably $R_2$ and $R_3$ are the same or different and each is methyl or ethyl or taken together with the nitrogen atom to which they are attached comprise a pyrrolidine, piperidine or morpholine ring. $—NR_2R_3$ is most suitably a dimethylamino group or a diethylamino group, and preferably a dimethylamino group.

Suitably $R_4$, which is attached at the 7- or 8-position, is hydrogen, halogen, $C_{1-4}$ alkyl, or trifluoromethyl. Most suitably $R_4$ is hydrogen, methyl, ethyl, chloro or fluoro. Preferably $R_4$ is hydrogen. When $R_4$ is other than hydrogen it is attached conveniently suitably at the 7-position.

Solvates of the compounds of the formula (I) are also included within the scope of the present invention. Preferred solvates include hydrates and $C_{1-4}$ alkanolates.

Salts of the compounds of formula (I) may be either acid additon salts or salts formed with the carboxylic acid group. Acid addition salts are preferred but salts formed from the carboxylic acid group may be particularly useful in preparing the corresponding carboxy compound. Pharmaceutically acceptable salts are preferred.

When used in medicine, the salts of the compund of formula (I) should be both pharmacologically and pharmaceutically aceptable, but non pharmaceutically acceptable salts may conveniently be used to prepare the free active compound or pharmaceutically acceptable salts thereof and are not excluded from the scope of this invention. Such pharmacologically and pharmaceutically acceptable acid addition salts include, but are not limited to, those prepared from the following acids: hydrochloric, sulphuric, nitric, phosphoric, maleic, salicylic, toluene-p-sulphonic, tartaric, citric, methanesulphonic, formic, malonic, isethionic, succinic, naphthalene-2-sulphonic and benzenesulphonic. Also, pharmaceutically acceptable salts can be prepared as alkaline metal or alkaline earth salts, such as sodium, potassium or calcium salts of the carboxylic acid group.

When the compounds of formula (I) contain a double bond in the side chain terminating in the carboxylic acid group, they exist in either the *cis* or *trans* isomeric form(s) (in relation to the aromatic ring). Both isomers and the isomeric mixture of these componds are included within the scope of the present invention. When $R_1CO_2H$ contains a double bond, the preferred isomers are those wherein the carboxylic acid group is *trans* to the aromatic ring.

Preferred compounds of the formula (I) include:—

trans-β-[10-2(2-dimethylaminopropyl)phenothiazin-2-yl]acrylic acid
[10-(2-dimethylaminopropyl)phenothiazin-2-yl]acetic acid
[10-(2-dimethylaminopropyl)phenothiazin-2-yl]propionic acid
10-(2-Dimethylaminopropyl)phenothiazine-3-carboxylic acid
*E*-3-(2-Carboxyvinyl)-10-(2-dimethylaminopropyl)phenothiazine
3-(2-Carboxyethyl)-10-(2-dimethylaminopropyl)phenothiazine
10-(2-Dimethylaminopropyl)phenothiazine-4(or 1)-carboxylic acid
(*E*)-4(or 1)-(2-Carboxyvinyl)-10-(2-dimethylaminopropyl)phenothiazine
4-(or 1)-(2-Carboxyethyl)-10-(2-dimethylaminopropyl)phenothiazine
[7-Chloro-10-(2-dimethylaminopropyl)-2-phenothiazinyl]acetic acid
[10-2-Dimethylaminoethyl)-7-methyl-2-phenothiazinyl]acetic acid
[10-(2-Dimethylaminoethyl)-7-fluoro-2-phenothiazinyl]acetic acid
2-[10-(2-Dimethylaminopropyl)-2-phenothiazinyl]-2-methylpropionic acid
2-[10-(3-Dimethylaminopropyl)-2-phenothiazinyl]-2-methylpropionic acid
2-Methyl-2-[10-[3-(N-pyrrolidino)propyl]-2-phenothiazinyl]propionic acid
10-(2-Dimethylaminopropyl)phenothiazine-2-carboxylic acid

or salts thereof.

Pharmacokinetic studies comparing the relative distribution in brain and plasma of two of the compounds of this invention and promethazine indicate that, unlike promethazine, these compounds (Compounds A and B see examples) do not readily penetrate the brains of rodents.

The present invention also provides an analogy method for preparing compounds of formula (I), which comprises:

a) The reaction of a compound of the formula (III)

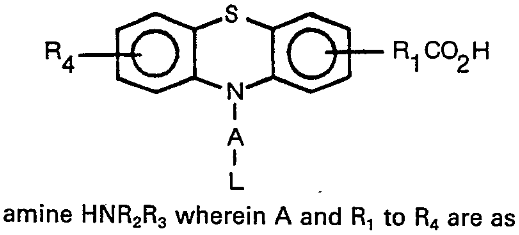

(III)

or an ester thereof with an amine $HNR_2R_3$ wherein A and $R_1$ to $R_4$ are as hereinbefore defined and L is a leaving group;

b) When it is required to prepare a compound of the formula (I) wherein $R_1$ is $(CH_2)_0$ (i) the reaction of a compound of the formula (IV):

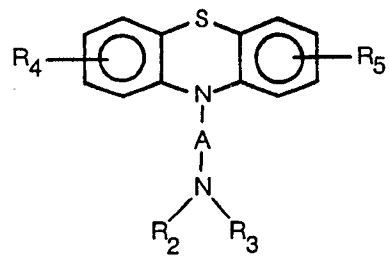

(IV)

wherein $R_2$, $R_3$, $R_4$ and A are as hereinbefore defined and $R_5$ is a hydrogen or halogen atom, with a $C_{1-6}$ alkyl lithium compound followed by treatment with carbon dioxide;

(ii) the oxidation of a compound of the formula (V):

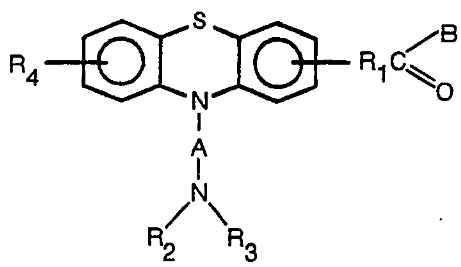

(V)

wherein $R_2$, $R_3$, $R_4$, and A are as hereinbefore defined, $R_1$ is a single bond and B is hydrogen;

c) When it is required to prepare a compound of the formula (I) wherein $R_1$ is $(CH_2)_a$ $CH=CH(CH_2)_b$ and a is 0 and b is 0 to 5 the reaction of a compound of the formula (V) as hereinbefore defined, with a Wittig reagent suitable for attaching the side chain $CH=CH(CH_2)_bCOR_6$, wherein $COR_6$ is an acid, ester or amide group as hereinbefore defined, followed by deprotection of the carboxy group;

d) When it is required to prepare a compound of the formula (I) wherein $R_1$ is $CH=CH$, the reaction of a compound of the formula (V) as hereinbefore defined, with malonic acid or a malonic acid ester;

e) the hydrolysis of a compound of the formula (VI):

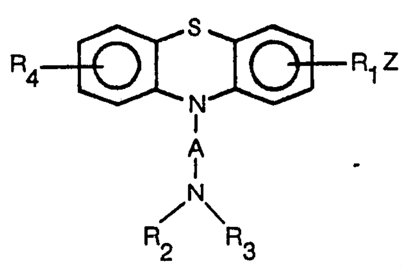

(VI)

wherein $R_2$ to $R_4$ and A are as hereinbefore defined and $R_1$ Z is a group $R_1$ CN wherein $R_1$ is a $C_{1-7}$ bivalent hydrocarbon group, or a group $R_7$ — $CH=C(Y)XH$ wherein $R_7$ is a $C_{1-6}$ bivalent hydrocarbon group or a single bond, X is oxygen or sulphur and Y is a secondary amino group;

f) the alkylation of a compound of the formula (VII):

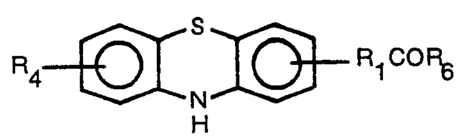

(VII)

wherein $R_1$ and $R_4$ are as hereinbefore defined, $COR_5$ is an ester or amide group, followed by deprotection and thereafter, optionally converting one compound of the formula (I) to another compound of the formula (I) by methods well known to those skilled in the art, for example the reduction of one or more double bonds.

The analogy methods are carried out suitably in the following manner:

a) Suitably leaving group L in the compounds of the formula (III) are those as defined by J. March, *Advanced Organic Chemistry*, 2nd ed., pages 683 and 895, McGraw Hill, New York, 1977, *e.g.* -Br, -Cl, toluene sulphonate, methane sulphonate, acyloxy (such as acetate), etc.

This reaction will normally be carried out in a solvent suitable for carrying out such displacement

reactions, for example a polar solvent, such as a $C_{1-4}$ alkanol or a polar aprotic solvent such as DMSO, at a temperature between 0 and 180°C.

The compounds of the formula (III) may be prepard by the reaction of an ester of the corresponding compound where L is a hydroxy group with an acid or a suitable reactive acid derivative, followed by removal of the ester function if desired. Suitable reactants include hydrogen halides, halogenated phosphorus compounds such as phosphorus pentachloride or phosphorus oxychloride, a suitable sulphonyl chloride (such as methane sulphonyl chloride or p-toluene sulphonyl chloride) or an acid anhydride, such as acetic anhydride. The reaction will conveniently be carried out in a suitable solvent under conditions well known to those skilled in the art, for example a non-protic solvent such as an ether or a halogenated hydrocarbon, in the presence of a base such as a tertiary amine (for example triethylamine) at a non-extreme temperature, for example between 0° and 100°C and conveniently at room temperature. When a tertiary amine is used as a base, an excess of this may be used as the solvent.

The hydroxy compounds may be prepared by reacting phenothiazines unsubstituted at the 10-position (i.e. by the group AL) with either alkylene oxides such as ethylene (or higher) oxide, or with an alkanol which also bears a leaving group, in the presence of a strong base, such as an alkali alcoholate, at a non-extreme temperature, for example between 0° and 100°C.

b)(i) The reaction of a compound of the formula (IV) with alkyl lithium followed by treatment with carbon dioxide is suitably carried out in a solvent inert under the reaction conditions utilised, for example benzene, toluene or an ether such as tetrahydrofuran, under an inert atmosphere, such as nitrogen, at a low temperature, for example −80°C and −20°C. $R_5$ is preferably bromine or iodine. The alkyl lithium compound is suitably butyl lithium. The reaction is conveniently carried out in toluene or tetrahydrofuran at a temperature between −80°C and −50°C under nitrogen. The compound of the formula (IV) may be prepared by the reaction of a compound of the formula (VIII):

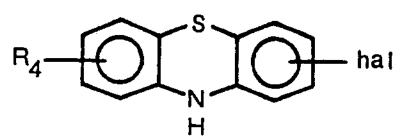

(VIII)

wherein $R_4$ is as hereinbefore defined with a compound $LANR_2R_3$ wherein A, $R_2$, $R_3$ and L are as hereinbefore defined. This reaction is suitably carried out in the presence of a base, such as an alkali metal alkoxide, for example potassium butoxide, in an inert solvent, for example xylene, at an elevated temperature, i.e. between 20° and 150°C and conveniently between 50° and 80°C.

(ii) The compound of formula (V) is suitably prepared by oxidatioon of the corresponding alcohol under conditions that will not lead to oxidation of the components of the phenothiazine ring, for example by oxidation with silver carbonate on diatomaceous earth in an inert hydrocarbon solvent, such as benzene at a non-extreme temperature, for example between 0° and 100°C, conveniently under reflux. The alcohol may be prepared by reduction of the corresponding acid or its ester, i.e. a compound of the formula (I) wherein $R_1$ is $(CH_2)_0$. This reduction may suitably be carried out using a metal hydride, such as lithium aluminium hydride, in an inert solvent, such as an ether, for example diethyl ether, at between 0 and 75°C and suitably under reflux.

This reaction is a conventional Wittig reaction and, as such, is analogous to those described in *Organic Reactions, 14,* 270—490 (1965) and *Pure and Applied Chemistry, 9,* 245—254 (1964). The reaction is suitably carried out in an anhydrous solvent inert under the reaction conditions utilised, for example toluene, benzene, tetrahydrofuran, dioxane, glycol ethers and $C_{1-6}$ alkyl ethers such as ethyl ether, at a temperature between −80°C and 100°C. The Wittig reagent will normally be prepared by treatment of a phosphonium salt with a strong base for example a $C_{1-4}$ alkyl or aryl lithium compound such as butyl lithium, or a metal hydride, such as sodium hydride in a suitable inert solvent, such as those specified above.

c) The Wittig reagent in reaction (c) is conveniently prepared by reacting a compound of the formula $(R_8)_2\,PO{\cdot}(CH_2)_dCO_2R_6$, wherein $R_6$ is as hereinbefore defined, $R_8$ is a $C_{1-4}$ alkoxy group and d is 1 to 6, or a compound of the formula $(R_9)_3P(CH_2)_dCO_2R_6$ wherein $R_6$ is as hereinbefore defined $R_9$ is $C_{1-4}$ alkyl or phenyl and d is 1 to 6 with a strong base, such as sodium hydride in a suitable inert solvent, such as tetrahydrofuran or dimethoxyethane at a temperature between 0° and 50°C, conveniently at room temperature.

The reaction between the Wittig reagent and compound of the formula (V) is conveniently carried out by adding the compound of the formula (V) to the Wittig reagent at a temperature of between 0° and 50°C and conveniently at room temperature.

d) The reaction of a compound of the formula (V) with malonic acid or an ester thereof is suitably carried out in the presence of a base, conveniently pyridine, which can also act as solvent at an elevated temperature i.e. between 20° and 150°C and conveniently between 80° and 120°C.

e) The hydrolysis of a compound of the formula (VI) is suitably carried out by the addition of a dilute mineral acid, for example hydrochloric acid and heating at an elevated temperature, for example between 50° and 100°C and conveniently at 100°C. Such hydrolysis reactions of nitrile groups, amide and thioamide groups are well known to those skilled in the art. The compounds of the formula (VI) wherein $R_1$ Z is a group $R_1$ CN may be prepared by the reaction of a compound of the formula (V) wherein B is hydrogen or methyl

and $R_1$ is a single bond or a $C_{1-6}$ bivalent hydrocarbon group as hereinbefore defined, with a compound $L_1CH_2N{=}C$ wherein $L_1$ is a sulphonyloxy group, for example tosyl in the presence of a base, for example an alkali metal alkoxide such as potassium butoxide, in an aprotic solvent such as dimethyl sulphoxide, at a non-extreme temperature, for example −20° to 100°C and conveniently 20°C. The compounds of the formula (VI) wherein $R_1$ Z is a group $R_7$—CH=C(Y)XH wherein X is a sulphur and Y is a secondary amino group may be prepared by the reaction of a compound of the formula (V) wherein $R_1$ is a single bond and B is hydrogen or $C_{1-4}$ alkyl with sulphur and a secondary amine, such as morpholine, at an elevated temperature, i.e. 50° to 150°C, preferably in an inert atmosphere. The corresponding amides (i.e. those compounds wherein X is oxygen may be prepared by hydrolysis of the corresponding thioamide or nitrile.

f) The alkylation of a compound of the formula (VII) is carried out under conditions well known to those skilled in the art of such reactions. The alkylating agent $LANR_2R_3$ (wherein L, A, $R_2$ and $R_3$ are as hereinbefore defined), is reacted with the compound of the formula (VII) in the presence of a base at a non-extreme temperature, for example between 0°C and 150°C in an inert solvent.

A strong base is conveniently utilised, for example an alkoxide such as t-butoxide, a hydride such as sodium hydride or sodamide. The temperature is conveniently between 20° and 90°C and the solvent is suitably a hydrocarbon, such as toluene, an ether, such as tetrahydrofuran, or a dipolar aprotic solvent, such as dimethylformamide or dimethylsulphoxide.

The compounds of the formula (VII) may be prepared as described in Scheme 1 attached hereto.

The reduction of one to two double bonds, i.e. the reduction of the double bond in the carboxy side chain may conveniently be carried out by hydrogenation in the presence of a transition metal catalyst, for example platinum or charcoal. The preparation of esters or amides from the corresponding carboxylic acid, and vice versa, may similarly be carried out by methods well known to those skilled in the art.

The compounds of this invention having antiallergic activity may be used for the same indications as clinically used antiasthmatic compounds, namely to help to control broncho-constriction and bronchospasm characteristic of allergic asthma and exercise induced asthma and the symptoms of broncho-constriction and bronchospasm resulting from acute or chronic bronchitis. The compounds are believed to inhibit the release of autocoids (i.e. histamine, serotonin and the like) from mast cells and to inhibit directly the antigen-induced production of histamine. Thus, they may be classified as mast cell stabilizers with antihistaminic action.

The compounds of this invention having antihistamine activity may be used for the same indications as clinically used antihistamines, namely to relieve detrimental symptoms (caused by histamine release) of nasal stuffiness due to colds and vasomotor rhinitis and for the symptomatic control of allergic conditions including nasal allergy, perennial rhinitis, urticaria, angioneurotic oedema, allergic conjunctivitis, food allergy, drug and serum reactions, insect bites and stings and desensitizing reactions. The compound may also be used in conditions responsive to its antipruritic activity including allergic dermatoses, neurodermatitis, anogenital pruritus, and pruritus of non-specific origin such as eczema, and of specific cause such as chickenpox, photosensitivity and sunburn. The present invention therefore provides a method for the symptomatic treatment of allergic conditions by the administration of an effective amount of a compound of the formula (I). The compounds of the present invention have been found to be substantially free from sedative effects and to have little or no anticholinergic effects.

The amount of active compound required for use in the above conditions will vary with the compound chosen, the route of administration and the condition and mammal undergoing treatment, and is ultimately at the discretion of the physician. A suitable oral dose of the active compound for a mammal is in the range of from 0.0003 to 1.0 mg per kilogram body weight per day; preferably from 0.04 to 0.24 mg/kg. For example a typical dose for a human recipient of compound (B) (see example 2 and Table 1 hereafter) is between 0.03 and 0.1 mg/kg body weight per day.

The desired daily dose is preferably presented as from one to six sub-doses administered at appropriate intervals throughout the day as needed. Where three sub-doses of compounds of formula (I) are employed, each will preferably lie in the range of from 0.014 to 0.08 mg/kg body weight; for example, a typical sub-dose of such a compound for a human recipient is between 1 and 20 mg, for example 4 or 8 mg.

Whilst it is possible for a compound of the formula (I) to be administered alone as the raw chemical, it is preferable to present the compound of formula (I) as a pharmaceutical formulation. Thus, the present invention also provides pharmaceutical formulations, both for veterinary and for human medical use, which comprise a compound of the formula (I) together with one or more pharmaceutically acceptable carriers thereof and optionally any other therapeutic ingredients. For example, the active compound may be formulated with a sympathomimetic agent such as the decongestant pseudoephedrine, an antitussive such as codeine, an analgesic, an antiinflammatory, an antipyretic, or an expectorant. The carrier(s) must be pharmaceutically acceptable in the sense of being compatible with the other ingredients of the formulation and not deleterious to the recipient thereof.

The formulations include those suitable for oral, rectal, topical, nasal, ophthalmic or parenteral (including subcutaneous, intramuscular and intravenous) administration.

The formulations may conveniently be presented in unit dosage form and may be prepared by any of the methods well known in the art of pharmacy. All methods include the step of bringing the active compound into association with a carrier which constitutes one or more accessory ingredients. In general, the formulations are prepared by uniformly and intimately bringing the active compound into association

with a liquid carrier or a finely divided solid carrier or both and then, if necesary, shaping the product into desired formulations.

Formulations of the present invention suitable for oral administration may be presented as discrete units such as capsules, cachets, tablets or lozenges, each containing a predetermined amount of the active compound (defined herein as a compound of formula (I)); as a powder or granules; or a suspension in an aqueous liquid or nonaqueous liquid such as a syrup, and elixir, an emulsion or a draught. A tablet may be made by compression or molding, optionally with one or more accessory ingredients. Compressed tablets may be prepared by compressing in a suitable machine, with the active compound being in a free-flowing form such as a powder or granules which is optionally mixed with a binder, disintegrant, lubricant, inert diluent, surface active agent or dispersing agent. Molded tablets comprised of a mixture of the powdered active compound with any suitable carrier may be made by molding in a suitable machine.

A syrup may be made by adding the active compound to a concentrated, aqueous solution of a sugar for example sucrose to which may also be added any accessory ingredient(s). Such accessory ingredient(s) may include flavourings, an agent to retard crystallization of the sugar or an agent to increase the solubility of any other ingredient, such as a polyhydric alcohol, for example glycerol or sorbitol, and suitable preservatives.

Formulations for rectal administration may be presented as a suppository with a usual carrier such as cocoa butter, or hydrogenated fats or hydrogenated fatty carboxylic acids.

Formulations suitable for parenteral administration conveniently comprise a sterile aqueous preparation of the active compound which is preferably isotonic with the blood of the receipient.

Nasal spray formulations comprise purified aqueous solutions of the active compound with preservative agents and isotonic agents. Such formulations are adjusted to a pH and isotonic state compatible with the nasal mucous membranes.

Ophthalmic formulations are prepared by a similar method to the nasal spray except that the pH and isotonic factors are adjusted to match that of the eye.

Topical formulations comprise the active compound dissolved or suspended in one or more media such as mineral oil, petroleum, polyhydroxy alcohols or other bases used for topical pharmaceutical formulations. The addition of other accessory ingredients, *vide infra,* may be desirable.

In addition to the aforementioned ingredients, the formulations of this invention may further include one or more accessory ingredient(s) selected from diluents, buffers, flavouring agents, binders, disintegrants, surface active agents, thickeners, lubricants, preservatives (including antioxidants) and the like.

The present invention also provides the first use of the compounds of the formula (I) in medicine.

The following examples are provided by the way of illustration of the present invention and should in no way be construed as a limitation thereof. All temperatures indicated are in degrees Celsius.

## Example 1

Trans-β-[10-(2-Dimethylaminopropyl)phenothiazin-2-yl]acrylic acid

a) 2-Bromo-10-(2-dimethylaminopropyl)phenothiazine (Compound ex-1a)

2-Bromophenothiazine (5.0 g) and potassium t-butoxide (2.4 g) were combined in 25 ml dry xylene and stirred under nitrogen and heated at 65°C for 10 minutes. To this was added a solution of 2-dimethylaminopropyl choride (2.8 g) in xylene (2 ml) dropwise over 10 minutes. Stirring under nitrogen and heating at 60—70° were continued for 3 hours. The reaction was poured into ethyl acetate (100 ml) and extracted with dilute HCl. The acid extract was basified with excess aqueous ammonia and extracted with $CH_2Cl_2$, which was dried ($MgSO_4$) and evaporated, leaving a viscous oil (6.35 g). The oil was a mixture of the title compound and its isomer 2-Bromo-10-(2-dimethylamino-1-methylethyl)phenothiazine, from which it was separated as a viscous colourless oil by preparative HPLC on a silica column, eluting with $EtOAc/CH_2Cl_2$ (4:1).

Compound *ex-1a* did not crystallize and no attempt was made to obtain a crystal salt. It was characterized by its nmr spectrum, which was consistent with its indicated structure and different from that of its isomer.

b) 10-(2-Dimethylaminopropyl)phenothiazin-2-yl-carboxaldehyde (Compound ex 1b)

Using syringes and a nitrogen atmosphere THF (20 ml) was added to a flask containing compound *ex-1a* (2.8 g). The solution was stirred and cooled in a dry ice/acetone bath, and n-butyl lithium (5.2 ml of a 1.5 M solution in hexane) was added dropwise over five minutes. After stirring at −78°C for 30 minutes, distilled DMF (0.8 ml) was added and the reaction was allowed to warm slowly (over 25 minutes) to ambient temperature. It was then poured onto cold dilute HCl which was washed with benzene and then basified with $NH_4OH$. The product was extracted into $CH_2Cl_2$ which was dried ($MgSO_4$) and evaporated, leaving 2.5 g of very viscous yellow oil, compound *ex-1b*.

Compound ex-1b did not crystallize and no attempt was made to obtain crystalline salts. It was characterized by its nmr and ir spectra, which were consistent with the structure indicated. The tlc showed a single component on silica ($CHCl_3/MeOH$, 9:1) which was slightly less mobile than starting material (compound *ex-1a*).

c) trans-β-[10-(2-Dimethylaminopropyl)phenothiazin-2-yl]acrylic acid (Compound A)

Compound *ex-1b* (2.4 g), malonic acid (1.1 g), and morpholine (0.120 g) were dissolved in pyridine (8 ml) and heated on steam. Carbon dioxide gas evolved briskly at first. After 2 hours, the pyridine was evaporated at reduced pressure. The residue was triturated with boiling EtOH (25 ml), which produced a yellow solid upon cooling and stirring. This was recrystallized from EtOH, yielding Compound *A*, yellow crystalline solid, mp 214—8°. The nmr and CHN analysis were consistent with the structure indicated.

Example 2

[10-(2-Dimethylaminopropyl)phenothiazin-2-yl]acetic acid

a) 2-acetylphenothiazine ethylene ketal (Compund ex-2a)

Under nitrogen a solution of 2-acetylphenothiazine (100 g), ethylene glycol (50 g), and p-toluenesulfonic acid hydrate (2.0 g) in benzene (900 ml) was refluxed under a water separator (Dean-Stark trap) for 18 hours. Analysis by tlc (silica gel, hexane/EtOAc 4:1) confirmed that almost all the starting compound was consumed and replaced by one with a higher Rf value (ex-4). After cooling, the benzene solution was washed with 1% NaOH solution, dried over solid $K_2CO_3$, and evaporated, leavinag 115 g of dark oil which froze to a dark green crystalline solid upon standing at ambient temperature. The nmr was consistent with the assigned structure. It was not purified further but was carried on to the next step.

b) 2-Acetyl-10-(2-Dimethylaminopropyl)phenothiazine (Compound ex-2b)

Crude compound *ex-2a* (47.5 g) and potassium t-butoxide (20.9 g) were combined in benzene (250 ml) and stirred under nitrogen for 15 minutes. To this was added 2-dimethylaminoisopropyl chloride (23 g), and the resulting solution was heated at reflux for 75 minutes. After cooling the amines were extracted into dilute aqueous HCl, and the resulting acid solution was heated for 20 minutes on steam to hydrolyze the ketal. After cooling the aqueous solution was washed with ether and then basified with excess NaOH. The free amines were then extracted into $CH_2Cl_2$, which was dried (MgSO) and evaporated to leave a red viscous oil which consisted of a crude mixture of compound *ex-2b* and its isomer, 2-acetyl-10-(2-dimethylamino-1-methylethyl)phenothiazine. Compound *ex-2b* is predominant and was obtained analytically pure by formation of the hydrogen maleate salt in EtOAc/MeOH and two recrystallizations from the same solvent, mp 187—91°. The nmr and CHN analysis were consistent with the structure indicated. The isomer was obtained pure from the mother liquors by column chromatography of the free amine on silica gel, eluting with EtOAc/EtOH (20:1).

c) [10-(2-Dimethylaminopropyl)phenothiazin-2-yl]acetic acid (Compound B)

Compound *ex-2b* (15.4 g), sulfur (3.2 g) and morpholine (10 ml) were combined and heated at reflux under nitrogen for 5 hours. The warm reaction was then poured into EtOAc (300 ml) plus ether (100 ml), which was stirred and filtered. A small amount of insoluble material was discarded. The amines were extracted into dilute HCl (400 ml $H_2O$ plus 16 ml conc. HCl). The aqueous solution was washed with ether and then basified with excess NaOH. The product wsa extracted into $CHCl_3$ (400 ml), which was washed with $H_2O$ (3 × 600 ml). Evaporation of the $CHCl_3$ solution produced a viscous red oil (16.4 g) whose tlc (silica gel, $CH_2Cl_2$/MeOH, 25:1) indicated many components present. The sample was fractionated by preparative HPLC, using two silica columns in sequence, eluting with $CH_2Cl_2$/MeOH (98:2), then switching to $CH_2Cl_2$/MeOH (97:3). The main fractions, after evaporation, yielded a total of 9.2 g of viscous orange oils, whose nmr's and tlc's indicated that they were imperfectly separated mixtures of the expected thiomorpholide and (apparently) its partial hydrolysis product, the oxymorpholide. Since both were satisfactory intermediates for hydrolysis to the desired acid, compound *B*, they were not purified further but were combined, dissolved in 4N HCl (225 ml) and refluxed 6 hours, which removed all trace of starting materials from the tlc.

The reaction was evaporated to a viscous green residue, which was dissolved in $H_2O$ (70 ml) and washed with EtOAc. The solution was then basified with a slight excess (litmus) of solid NaOH, and washed with $CH_2Cl_2$. Partial evaporation of the aqueous solution gave precipitation of the Na salt of compound *B*. The precipitation was increased by dilution with an equal volume of saturated NaCl solution. The resulting solid was filtered and recrystallized from $H_2O$/sat NaCl (4:1). The product after filtration and vacuum drying at 70°C overnight, was 4.3 g of dry powder, which gave correct CHN analysis and Karl Fischer analysis for Compound *B*, Na salt, with 5.5 moles $H_2O$ included.

A hydrogen maleate salt was also prepared from this, mp 115—120°, whose nmr and elemental analysis were correct for the structure indicated.

Example 3

[10-(2-Dimethylaminopropyl)phenothaizin-2-yl]propionic acid

a) [10-(2-Dimethylaminopropyl)phenothiazin-2-yl]propionitrile Compound ex-3a.

Under nitrogen a solution of tosylmethyl isocyanide (4.0 g) in DMSO (15 ml) and THF (4 ml) was stirred in an ice bath, and potassium t-butoxide (5.3 g) was added. After five minutes 0.68 g of methanol was added. To this was then added dropwise over three minutes a solution of compound *ex-2b* (3.4 g) in DMSO (3 ml) and THF (1 ml). It was allowed to warm to ambient temperature and was stirred for 20 hours. The reaction was poured into ether and washed with $H_2O$. The amines were extracted into dilute HCl, which was

washed with ether and then basified with excess NaOH. The free amines were then extracted into ether, which was dried ($MgSO_4$) and evaporated, leaving the crude product as an oil (2.5 g) whose tlc (silica, $CH_2Cl_2$/MeOH, 24:1) showed multiple components. The desired component, compund *ex-3a*, was separated cleanly by preparative HPLC on silica, eluting with $CH_2Cl_2$/MeOH (97:3). Compound *ex-3a* was an oil and was identified by its ir and nmr spectra, which were consistent with the structure assigned.

b) [10-(2-Dimethylaminopropyl)phenothiazin-2-yl]propionic acid (Compound C).

Compound *ex-3a* (1.7 g) was dissolved in 4N HCl (50 ml) and heated at reflux for 20 hours. The solution was evaporated at reduced pressure to a viscous oil which was then redissolved in $H_2O$ (30 ml), washed with ether, and then brought to a basic pH (⅓10, litmus) by careful addition of NaOH. The resulting solution was washed with ether and then evaporated to about half the original volume. The HCl salt of compound *C* was precipitated as an oil by addition of excess concentrated HCl. This oil was redissolved in $H_2O$, reprecipitated (again as an oil) with excess HCl, and then rubbed with ether, which caused the oil to solidify. This salt was recrystallized from MeOH/EtOAc to yield *C*·HCl·$H_2O$, colourless crystals, mp 156—62°C (dec.), whose elemental analysis (CHN) was correct for the formula assigned.

Example 4

10-(2-Dimethylaminopropyl)phenothiazine-3-carboxylic acid

To a solution of 3-bromo-10-(2-dimethylaminopropyl)phenothiazine (2.5 g) (prepared in a similar manner to the 2-bromo compound in Example 1) in dry ether, cooled at 0°C, was added dropwise a solution of butyl lithium in hexane (6.7 ml of 1.55 M solution). The solution was stirred at 0°C for 1 hour, then cooled to −40°C and a large excess of solid carbon dioxide was added. The mixture was allowed to come to room temperature and water was added. After thorough mixing, the ether layer was separated and discarded, the aqueous solution was brought to pH 6 by careful addition of 2N-hydrochloric acid, and then evaporated to dryness. The residue was boiled with ethanol and the filtered ethanol solution was evaporated to dryness to leave a semi-solid mass. Chromatographic separation of the material on a column of silica with mixtures of chloroform and methanol as eluant gave the impure carboxylic acid as a grey-coloured solid (1 g) which was further purified by way of its methyl ester, as follows.

A solution of crude acid (670 mg) in methanol (20 ml) and concentrated sulphuric acid (0.65 ml) was refluxed for 3 hours. Most of the methanol was evaporated and water was added. The solution was washed with ether, basified with 2N-sodium hydroxide solution and the precipitated oil was extracted into ether. The ether solution was washed with water, dried ($MgSO_4$) and evaporated to leave yellow viscous oil (570 mg). This ester was dissolved in a small excess of dilute hydrochloric acid, the solution was evaporated to dryness, and the residue was recrystallised from a mixture of ethanol and ether to give fine pale yellow needles of methyl 10-(2-dimethylaminopropyl)phenothiazine-3-carboxylate hydrochloride, m.p. 218°C (decomp).

The foregoing ester hydrochloride (584 mg) in a mixture of ethanol (4 ml) and 2*N*-sodium hydroxide solution (1.77 ml) was stirred at room temperature for 20 hours. The solution was neutralised by addition of 2*N*-hydrochloride acid (1 ml) and evaporated to dryness. The residue was extracted with hot ethanol and the filtered extract was evaporated to dryness. The residaul solid was recrystallised twice from methanolethyl acetate to give small colourless prisms, m.p. 185—188°C, of 10-(2-dimethylaminopropyl)phenothiazine-3-carboxylic acid (Compound D).

Example 5

E-3-(2-Carboxyvinyl)-10-(2-dimethylaminopropyl)-phenothiazine

a) (E)-3-(2-methoxycarbonylvinyl)-10-(2-dimethylaminopropyl)phenothiazine.

To a stirred solution of 3-bromo-10-(2-dimethylaminopropyl)phenothiazine (4.0 g) in dry ether at 0°C was added dropwise a solution of butyl lithium (8.5 ml of 1.55 *M* solution in hexane). The resulting yellow solution was stirred at 0°C for one hour and then treated dropwise, during 1 hour, with a solution of dimethylformamide (1.7 ml) in dry ether (20 ml). The reaction mixture was stirred at room temperature for 3 hours, then shaken with water and ether. The washed and dried ethereal solution was evaporated to leave the crude aldehyde as a yellow oil.

A solution of this crude aldehyde (4.0 g) in dry 1,2-dimethoxyethane (20 ml) was added to a stirred solution of the phosphonate carbanion produced from diethyl methoxycarbonylmethylphosphonate (2.69 g) and sodium hydride (385 mg of 80% dispersion in oil) in 1,2-dimethoxyethane (30 ml) at room temperature. After 4 hours' stirring the reaction mixture was evaporated *in vacuo* and water and ether were added. The ethereal phase was separated, washed, dried and evaporated to leave crude (*E*)-3-(2-methoxycarbonylvinyl)-10-(2-dimethylaminopropyl)phenothiazine as a fluorescent yellow oil.

b) E-3-(2-carboxyvinyl)-10-(2-dimethylaminopropyl)phenothiazine.

This ester (4 g) was hydrolysed in a solution of ethanol (22 ml) and 2*N*-sodium hydroxide solution (8.8 ml) at room temperature for 4 hours. The product was isolated as described in Example 1 to give the crude carboxylic acid as an orange coloured gum (3.0 g). This was dissolved in warm 0.4 *N*-hydrochloric acid and concentrated hydrochloric acid was added to induce crystallisation of orange coloured plates. Recrystallisation from a mixture of ethanol and ether gave small yellow needles of *E*-3-(2-carboxyvinyl)-10-(2-dimethylaminopropyl)phenothiazine hydrochloridehemihydrate, m.p. *ca* 170°C (efferv). After drying at 150°C, the anhydrous salt had m.p. 220—225°C (decomp.) (Compound E).

Example 6

3-(2-carboxyethyl)-10-(2-dimethylaminopropyl)phenothiazine

A solution of *E*-3-(2-carboxyvinyl)-10-(2-dimethylaminopropyl)phenothiazine hydrochloride (Example 4)(1g) in methanol (20 ml) containing 10% palladium-on-charcoal catalyst (100 mg) was stirred in an atmosphere of hydrogen until uptake was complete (9 hours). The catalyst was removed by filtration and the solvent was evaporated to leave the hydrochloride of the saturated carboxylic acid. It was freed from a small impurity by esterification as described in Example 3, chromatography of the ester on a column of silica in chloroform-methanol (50:1), and hydrolysis of the pure ester as described in Example 4. The 3-(2-carboxyvinyl)-10-(2-dimethylaminopropyl)phenothiazine so obtained crystallised from an ethanol-ether mixture as a cream-coloured solid, m.p. 170—173°C (Compound F).

Example 7

10-(2-Dimethylaminopropyl)phenothiazine-4(or 1)-carboxylic acid

To a stirred solution of 10-(2-Dimethylaminopropyl)phenothiazine (5.7 g) in dry ether (150 ml) was added dropwise at 20°C a solution of butyl lithium (20 ml of 1.55 *M* solution in hexane). The solution was stirred at this temperature for 30 hours, then cooled to −15°C and solid carbon dioxide (*ca* 25 g) was added. The mixture was allowed to come to room temperature and left to stand for 16 hours. Water was added, the mixture was well shaken and the ethereal layer was separated. Evaporation of the washed and dried extract yielded unchanged starting material (4.5 g). The aqueous solution was neutralised by addition of 2*N*-hydrochloric acid and evaporated to dryness. The residue was extracted with ethanol and the filtered extract was evaporated to dryness. The residue (2.5 g) was purified by chromatography on a column of silica with chloroform-methanol mixtures as eluants to give 10-(2-Dimethylaminopropyl)phenothiazine-4(or 1)-carboxylic acid, as colourless needles, m.p. 220—222°C (decomp.) (from methanol) (Compound G).

Example 8

(E)-4(or 1)-(2-carboxyvinyl)-10-(2-dimethylaminopropyl)phenothiazine.

a) (*E*)-4(or 1)-(2-methoxycarbonylvinyl)-10-(2-dimethylaminopropyl)phenothiazine 10-(2-Dimethylaminopropyl)phenothiazine (5.7) was treated with butyl lithium under the conditions described in Example 7. To the resulting solution was added gradually dimethylformamide (3.1 ml) and the mixture was allowed to stand at room temperature for 18 hours. The crude aldehyde was isolated by ether extraction and used without further purification.

The crude aldehyde was treated with the phosphonate carbanion derived from diethyl methoxycarbonylmethylphosphonate as described in Example 5 to give 4 (or 1)-(2-methoxycarbonylvinyl)-10-(2-dimethylaminopropyl)phenothiazine which was crystallized from ethanol, m.p. 129—130°C.

b) *E*-4(or 1)-(2-carboxyvinyl)-10-(2-dimethylaminopropyl)phenothiazine. This ester was hydrolysed with dilute NaOH solution as described in Example 5 to give E-4(or 1)-(2-carboxyvinyl)-10-(2-dimethylaminopropyl)phenothiazine, crystallising from isopropanol-light petroleum (b.p. 60—80°C) as small yellow needles, m.p. 120—125°C (Compound H).

Example 9

4-(or 1)-(2-carboxyvinyl)-10-(2-dimethylaminopropyl)phenothiazine

A solution of (*E*)-4(or 1)-(2-methoxycarboxyvinyl)-10-(2-dimethylaminopropyl)phenothiazine (500 mg) in ethyl acetate (20 ml) was stirred in an atmosphere of hydrogen in the presence of Raney nickel catalyst until no more hydrogen was absorbed. The filtered solution was evaporated to give 4-(or 1)-(2-methoxycarboxyethyl)-10-(2-dimethylaminopropyl)phenothiazine which was subjected to hydrolysis in aqueous ethanol sodium hydroxide solution as described in Example 5. The 4(or 1)-(2-carboxyvinyl)-10-(2-dimethylaminopropyl)phenothiazine so produced crystallised from isopropanol in colourless needles, m.p. 155—157°C (Compound I).

Example 10

[7-Chloro-10-(2-dimethylaminopropyl)phenothiazinyl]acetic acid Compound K

a) 4′-Bromo-3′-nitrophenylacetic acid (Compound ex-10a)

Concentrated $HNO_3$ (20 ml) and $H_2SO_4$ (20 ml) were mixed and 4′-bromophenylacetic acid (21.5 g) was added to the stirred solution in portions over 20 minutes with water cooling such that the internal temperature was maintained in the 50—55°C range. This mixture was then heated for 10 minutes at 85°. The mixture was then poured onto ice and diluted with $H_2O$. The resulting yellow solid was filtered, washed with $H_2O$, dissolved in a small excess of 2N NaOH, and then reprecipitated by addition of excess concentrated HCl. The solid was dried and recrystallised three times from benzene, yielding compound *ex-10a* (10.2 g) mp. 108—113°. NMR analysis was consistent with a single isomer of the structure indicated.

b) Ethyl-4′-bromo-3′-nitrophenylacetate (compund ex 10b)

Compound *ex-10a* was suspended in a solution of ethanol (400 ml), triethylorthoformate (25 ml) and

concentrated $H_2SO_4$ (2 ml) and heated at reflux for 3 hours. The solvent was evaporated and the residue was dissolved in ether and washed sequentially with $H_2O$, 5% NaOH, and saturated NaCl. The ether was dried and evaporated, leaving compound *ex-10b* (35.1 g), a mobile yellow-orange oil whose nmr was consistent with the structure indicated.

c) Ethyl-4′-(4-chlorophenylthio)-3′-nitrophenylacetate (Compound ex-10c)

A mixture of compound *ex-10b* (8.6 g), 4-chlorothiophenol (5.5 g) and $Na_2CO_3$ (6.3 g) in ethanol (75 ml) was stirred under nitrogen at reflux for 5½ hours. It was poured into ice-cold 5% NaOH (900 ml) and extracted with ether (3 × 100 ml). The ether was washed with 5% NaOH and then dried ($MgSO_4$) and evaporated, leaving compound *ex-10c* (10.3 g) as a yellow oil which froze to a solid (m.p. 71—82°), whose nmr was consistent with the structure indicated. It was not purified further.

d) Ethyl-(7-chloro-2-phenothiazinyl)acetate (Compound ex 10d)

Compound *ex-10c* (24.3 g) and triethyl phosphite (46.0 g), were combined in dried and deoxygenated n-propylbenzene (200 ml) and refluxed under $N_2$ for 4 hours. The volatile components were removed on the $H_2O$ aspirator at 80°. The dark residual oil was purified by flash column chromatography on silica, eluting with hexane (ethyl acetate) (6:1). This yielded 6.1 g of a yellow solid which was recrystallized from ethanol/ethyl acetate (1:1). The product, compound *ex-10d* was a colourless solid (m.p. 193—4°) whose nmr was consistent with the proposed structure. This cyclization/rearrangement reaction has been described by Cadogan, *et al., J. Chem. Soc* (1970) 2437.

e) Ethyl-[7-chloro-10-(2-dimethylaminopropyl)-2-phenothiazinyl]acetate (Compound ex-10e) (Compound J)

Compound *ex-10d* (4.1 g) and potassium t-butoxide (1.52 g) were combined in dry deoxygenated toluene (30 ml) and stirred under $N_2$ for 10 minutes. 2-Chloro-1-dimethylaminopropane (1.7 g) was added, and the reaction was heated at 80° for 1 hour. A further 1.5 g potassium t-butoxide and 1.7 g 2-chloro-1-dimethylaminopropane were added stirring and heating were continued for 2 hours. The reaction was diluted with toluene (50 ml) and washed with $H_2O$ and saturated, NaCl. The amines were then extracted into dilute HCl, which was washed once with toluene, basified (litmus, conc $NH_4OH$), and extracted with $CHCl_3$ yielded 5.85 g of an oil with numerous components (silica tlc, EtOH/EtOAc 6:1). This oil was fractionated by silica flash-column chromatography, eluting with EtOAc/EtOH (progressively 12:1, 8:1, 5:1). The title compound *ex-10e* (1.0 g) was an oil. A hydrogen maleate salt was made and crystallized from ethyl acetate, mp 158—160° (dec), whose nmr and CHN analyses were consistent with the structure indicated.

f) [7-Chloro-10-(2-dimethylaminopropyl)-2-phenothiazinyl]acetic acid (Compound *K*)

A solution of compound *ex-10e* (0.4 g) in conc. HCl (10 ml) and $H_2O$ (10 ml) was heated on steam for 16 hours. Evaporation of the solvent left a glassy residue which was dissolved in 2-propanol and decolourised (charcoal). Cooling the solution yielded the HCl addition salt as a nearly colourless solid, mp 140° (dec). The melting (decomposition) point was difficult to reproduce, as it depended strongly on the rate of heating. The compound was a monohydrate, ex-10F·HCl·$H_2O$, whose CHN, nmr and mass spectral analyses, were correct for this formula and structure.

Example 11

[10-(2-Dimethylaminoethyl)-7-methyl-2-phenothiazinyl]acetic acid. Compound M

a) Ethyl 4′-(4-methylphenylthio)-3′-nitrophenyl acetate Compund ex-11a

This was made by the method of Example 10c, except that 4-methylthiophenol was employed instead of 4-chlorothiophenol. The crude product compound ex-11a, was a yellow solid mp 78—82°, whose nmr spectrum was consistent with the proposed structure. It was taken for the next step without further purification.

b) Ethyl (7-methyl-2-phenothiazinyl)acetate. Compound ex-11b.

This was synthesized from compound *ex-11a* by the method of Example 10. The column chromatography and recrystallization were also the same. The product compound *ex-11b*, was a crystalline solid, mp. 189—192°, whose nmr was consistent with the indicated structure.

c) Ethyl[10-(2-dimethylaminoethyl)-7-methyl-2-phenothiazinyl]acetate. Compound ex-11c. (Compound L).

This was prepared by the procedure of Example 10d, using 2-dimethylaminoethyl chloride. The free base was purified by flash column chromatography, eluting with EtOH/EtOAc (1:10). The oil was converted to a hydrogen maleate salt and crystallized from EtOAc. This was a solid, compound *ex-11c* hydrogen maleate, mp 146—148°, whose nmr spectrum and elemental analysis were consistent with the structure indicated.

d) [10-(2-Dimethylaminoethyl)-7-methyl-2-phenothiazinyl]acetic acid. (Compound M).

Compound *ex-11c* (0.50 g) was dissolved in 3.5 N HCl (14 ml) and heated on steam for 10 hours. After evaporation to dryness the residue was taken into 2-propanol and decolourized (charcoal) and then reevaporated. The residue was rubbed with ether until a solid formed, which was partly purified by solution

in methanol and addition of ethyl acetate and ether. This produced a yellow powder whose HPLC chromatogram indicated significant contamination. Therefore it was purified by semi-preparative HPLC on a 10 mm by 50 cm. Spherisorb G.P. $C_{18}$ column, with Brownlee $C_{18}$ guard column eluting with 40% MeOH + 0.1% triethylamine and finally 100% MeOH + 0.1% triethylamine. This yielded the title compound as a neutral solid, mp 127—132° (dec), whose nmr and CHN analysis and mass spectrum were satisfactory although at least one unidentified contaminant was still apparent (tlc, nmr, and mass spectrum).

Example 12

[10-(2-Dimethylaminoethyl)-7-fluoro-2-phenothiazinyl]acetic acid. (Compound N)

a) Ethyl 4'-(4-fluorophenylthio)-3'nitrophenyl acetate. Compound ex 12a

This was made by the method of example 10c, using 4-fluorothiophenol. The crude product, compound *ex-12a* was a yellow solid mp 41—48° whose nmr was consistent with the structure indicated and was used without further purification.

b) Ethyl(7-fluoro-2-phenothiazinyl)acetate. Compound ex 12b.

This was synthesized from compound *ex-12a* by the method of example *10-d*. The recrystallised product, compound *ex-12b* mp 167—169°, had an nmr consistent with the structure indicated.

c) Ethyl [10-(2-diethylaminoethyl)-7-fluoro-2-phenothiazinyl]acetate. Compound ex-12c.

This compound was obtained as an oil by synthesis from compound *ex-12b* and 2-diethylaminoethyl chloride by the method of example 10d and purified by flash column chromatography on silica eluting with ether. Its nmr and mass spectrum were consistent with the structure indicated.

d) [10-(2-diethylaminoethyl)-7-fluoro-2-phenothiazinyl]acetic acid. Compound N.

Compound *ex-12c* (0.6 g) was suspended in a mixture of dioxane (3 ml) and 1N NaOH (6 ml) and the mixture was heated at reflux for 24 hours. This was evaporated, acidified with 1N HCl (8 ml) and evaporated to dryness. The residue was digested with hot 2-propanol (30 ml). Filtration and evaporation of solvent yielded the HCl salt of compound N as a resinous mass which failed to crystallize. It was therefore purified by HPLC using a 10 mm × 50 cm Spherisorb G.P. (reverse-phase) $C_{18}$ column and a Brownlee $C_{18}$ guard column, eluting with 50% MeOH plus 0.1% triethylamine. This yielded neutral compound N as a solid which, after recrystallization from ethyl acetate had mp = 141—143°. Its CHN analysis, nmr, and mass spectrum were consistent with the structure indicated.

Example 13

2-[10-(2-Dimethylaminopropyl)-2-phenothiazinyl]-2-methylpropionic acid Compound P

a) 2-(4-Bromophenyl)-2-methylpropionic acid. Compound ex 13a.

The ethyl ester of the title compound had been prepared by the method described in *J. Am. Chem. Soc., 93,* 6877—87 (1971). This ester (54.1 g) was refluxed in a solution of ethanol (50 ml) and NaOH (40 g), in $H_2O$ (400 ml) for 5 hours. The mixture was cooled to ambient temperature, washed with pentane and then acidified with concentrated HCl (100 ml). This precipitated compound *ex-13a* as a white powder, mp 121—124°, whose nmr was consistent with the structure indicated.

b) 2-(4-Bromo-3-nitrophenyl)-2-methylpropionic acid. Compound ex-13b.

This was obtained by nitration of compound *ex 13a* by the method of example 10a. The resulting compound *ex-13b* was a solid, mp 167—71°, whose nmr was consistent with the structure indicated.

c) Ethyl 2-(4-bromo-3-nitrophenyl)-2-methyl propionate. Compound ex-13c.

This ester was obtained from compound *ex-12b* by the method described in example 10b. The resulting compound *ex-13c* was a solid which melted just above room temperature. Its nmr was consistent with the structure indicated, and it was used without further purification.

d) Ethyl 2-methyl-2-(3-nitro-4-phenylthiophenyl)propionate. Compound ex-13d.

This synthesis was performed by the method of Example 10c using thiophenol and compound *ex-13c*. The product, compound *ex-13d*, was obtained in quantitative yield as a very viscous yellow oil which failed to solidify. Its tlc was essentially homogeneous and its nmr was consistent with the structure indicated, so it was used without further purification.

e) Ethyl 2-methyl-2-(2-phenothiazinyl)propionate Compound ex-13e

This compound was made from compound *ex-13d* by the cyclization method of Example 10d and purified to apparent homogeneity (tlc, silica, hexane/ethyl acetate 10:1) by flash column chromatography. It did not crystallize, but its nmr was consistent with the structure indicated, and it was used without further purification.

f) Ethyl 2-[10-(2-dimethylaminopropyl)-2-phenothiazinyl]-2-methylpropionate. Compound ex-13f. Compound O.

This compound was synthesized from compound *ex-13e* by the alkylation reaction of Example 10e. The free base was an oil from which a crystalline salt was made and crystallized from ethylacetate/hexane, compound *ex-13F* hydrogen maleate. $H_2O$, mp 107—110°. Its CHN, nmr, and mass spectral analyses were consistent with the structure indicated.

g) 2-[10-(2-Dimethylaminopropyl)-2-phenothiazinyl]-2-methylpropionic acid. Compound P.

This was synthesized by base (NaOH) hydrolysis of compound *ex-13f* by the method of Example 12d. The crude HCl salt obtained after acidification, evaporation, and 2-propanol digestion was successfully solidified by rubbing with ether and then was recrystallised from methanol/ethyl acetate to yield compound P HCl mp 192—196° (dec), whose CHN, nmr, and mass spectral analyses were consistent with the indicated structure.

Example 14

2-[10-(3-Dimethylaminopropyl)-2-phenothiazinyl]-2-methylpropionic acid. Compound Q.

a) Ethyl 2-[10-(3-dimethylaminopropyl)-2-phenothiazinyl]-2-methylpropionate. Compound ex-14a.

Compound *ex-13e* (2.9 g) was dissolved in dry dimethylformamide (30 ml), followed by KOBut (1.13 g) and LiI (0.20 g). This was heated to 80° under $N_2$, and 3-dimethylaminopropyl chloride (1.2 g, freshly released from its HCl salt) was added. After heating at 80° for 30 minutes another 0.24 g of KOBut and 0.7 g 3-dimethylaminopropyl chloride were added followed 30 minutes later by a final 0.34 g of KOBut. After 30 more minutes the reaction was poured into water and extracted with $CH_2Cl_2$. Upon evaporation the $CH_2Cl_2$ yielded 4.3 g of viscous oil which was flash-chromatographed on silica eluting with $CH_2Cl_2$/MeOH, 96:4. This produced 2.8 g of compound *ex-14a* as an oil which was apparently homogeneous by tlc and whose nmr spectrum was consistent with the structure indicated.

b) 2-[10(3-dimethylaminopropyl)-2-phenothiazinyl]-2-methylpropionic acid Compound Q

This compound was prepared by base (1N NaOH), hydrolysis of compound *ex-14a* by the method of Example 12d. The HCl salt crystallized from aqueous HCl as a hydrate, compound Q HCl. $H_2O$, mp 128° (dec; the melting point was not reproducible, as it depended strongly on the rate of heating). The CHN, nmr, and mass spectral analyses were consistent with the structure and formula indicated.

Example 15

Ethyl 2-methyl-2-[10-[3-(N-pyrrolidino)propyl]-2-phenothiazinyl]propionate. Compound R.

a) Ethyl 2-methyl-2-[10-(3-bromopropyl)-2-phenothiazinyl]propionate. Compound ex-15a.

Compound *ex-13e* (4.7 g) was dissolved in 1,3-dibromopropane (25 ml) and KOBut (1.67 g) was added. The mixture was stirred at ambient temperature under $N_2$ for 30 minutes and then was heated to 80°. After heating 30 minutes a further KOBut was added, and this addition was repeated one more time after heating another 30 minutes. After the last addition the reaction was heated another hour and then extracted between ether and $H_2O$. The ether layer was evaporated and the residue was partially purified by column chromatography on silica gel, eluting with hexane/ethyl acetate (20:1). This yielded the target compound (compound (*ex-15a*)) as an oil contaminated by a substantial amount of a faster-eluting compound, which was identified by nmr and mass spectrum as the olefin formed by elimination of HBr from Compound *ex-15a*. The mixture of compound *ex-15a* and its contaminant was characterised by its nmr and mass spectrum which were consistent with the structure indicated. Since the contaminant was expected to be innocuous in the subsequent reaction, the product mixture was taken from the next step without further purification.

b) Ethyl 2-methyl-2-[10-[3-(N-pyrrolidino)-propyl]-2-phenothiazinyl]propionate. Compound R.

Compound *ex-15a* (0.8 g) and pyrrolidine (1.7 g) were dissolved in ethanol (5 ml), and the solution was allowed to stand at ambient temperature for 5 days. The mixture was then extracted between ether and dilute HCl and the ether was washed with more HCl. The combined HCl solutions were then treated with excess NaOH and extracted with $CH_2Cl_2$. The $CH_2Cl_2$ was dried and evaporated and the residue was chromatographed on a short silica column, eluting with $CH_2Cl_2$/MeOH (94:6). This produced Compound R as a clear viscous oil which was homogeneous by tlc. Attempts to make a crystalline addition salt were unsuccessful, but the compound was characterized by its nmr and mass spectra, which were consistent with the structure indicated.

Example 16

10-(2-Dimethylaminopropyl)phenothiazin-2-yl-carboxylic acid (Compound S).

A silver oxide suspension was prepared by dissolving silver nitrate (357 mg) in $H_2O$ (1.0 ml) and adding this to 1.00 g of NaOH solution (4.20 molal). To the resulting suspension was added ethanol (2 ml) and 10-(2-dimethylaminopropyl)phenothiazin-2-yl-carboxyaldehyde (300 mg). This suspension was stirred at ambient temperature for 10 minutes and then at 60° for 10 minutes. The warm suspension was filtered and the filtrate was partially evaporated, which caused precipitation of the sodium salt of the desired carboxylic acid. This was collected and converted to its HCl addition salt with excess conc HCl. The HCl salt was

dissolved in ethanol from which it crystallized, yield *Compound S* hydrochloride as a solid m.p. 257—261° (dec). Its mass spectrum and elemental analysis were consistent with the structure indicated.

Example 17
Antihistamine Activity

A. *In vitro* antihistamine activity: The longitudinal muscle was isolated from the intact ileum of guinea-pigs (Hartley, male 250—400 g) and placed in an organ bath under 300 mg tension. After one hour of equilibration, cumulative concentration-response curves (Van Rossum, J. M., *Arch. Int. Pharmacodyn. Ther. 143,* 299—330, 1963) to histamine were obtained. Following washing, the tissues were incubated for one hour with the test compound and then a second histamine concentration-response curve was run. Shifts to the right of the agonist concentration-response curve produced by the antagonists were used to construct Schild plots (Arunlakshana, O. and Schild, H. O., *Br.* J. *Pharmacol. 14,* 48—58, 1959). Regression of Log (dr-1) on Log [B], where dr is an equiactive response in the presence and absence of antagonist and [B] is the molar concentration of antagonist, allowed an estimate of $pA_2$, i.e. the negative log of the concentration of antagonist which shifts the control histamine concentration response curve 2× to the right.

| Compound | $pA_2$ |
|---|---|
| Promethazine | 8.9* |
| A | 8.2 |
| B | 9.4 |
| C | 8.1 |
| D | 6.2 |
| E | 6.2 |
| F | 5.2 |
| G | 6.8 |
| H | 7.8 |
| I | 7.2 |
| J | 9.0 |
| K | 8.8 |
| L | 9.1 |
| M | 8.9 |
| N | 8.6 |
| O | 8.5 |
| P | 8.8 |
| Q | 8.7 |
| S | 9.0 |

* R. B. Barlow, *Introduction to Chemical Pharmacology,* 2nd. ed. p. 363, Wiley, New York, 1964.

B. *In vivo* Antihistaminic Acitivity: Guinea pigs (Hartley, male, 300—350 g) were fasted for 20 hours and then dosed p.o. or i.p. with the test compound. One hour after dosing, on an individual basis, the guinea pigs were place in a clear plastic chamber which was saturated and continually gassed with 0.25.% histamine from an aerosol nebulizer. The guinea pigs were monitored for signs of histamine anaphylaxis

(e.g. cough, sneeze, strong abdominal movements, cyanoses or loss of righting). Under the test conditions, control animals collapsed on average within 33 seconds. $ED_{50}$'s for protection against histamine were calculated by probit analysis. In this test the $ED_{50}$ indicates that at that particular dose 50% of the animals were completely protected against histamine challenge at the time of testing (1 hour post-dosing). Complete protection was defined as no histamine symptoms for six minutes in the aerosol chamber (approximately 10× the collapse time of the control animals).

TABLE 1
Results of Antihistamine Assays

| Compound | $ED_{50}$(mg/kg, p.o.) |
|---|---|
| Triprolidine | 5.77 |
| A | 1.7 |
| B | 0.3 (4 hours post dosing) |

In addition to these results, it was found that Compound A could provide very long durations of antihistaminic activity (e.g. 11 mg/kg p.o. is the $ED_{50}$ for 24 hours protection).

Anaphylactoid Testing Procedure

Non-fasted, Wister rats (180—300 g) were dosed with the test compound, (i.p. or p.o.) 2 hours before compound 48/80 challenge. One hour prior to challenge, 5 mg/kg i.p. of propranolol was administered. The anaphylactoid inducing agent, compound 48/80, was given intravenously at 2 mg/kg and the animals were monitored for symptoms of respiratory distress.

Data were analyzed by Probit determinations. The response was quantitated by determining the dose of test compound which protected 50% of the animals from death at a given time point.

The above experimental design does not give positive results for selective antihistamines. Also rats do not respond to histamine (i.v.) with symptoms of anaphylaxis. Agents which block the effects of compound 40/80 are commonly classified as inhibitors of anaphylactic mediators or inhibitors of the release of anaphylic mediators.

Inhibition of Compound 48/80 Inducted Anaphylactoid Reaction

| Compound | $ED_{50}$* |
|---|---|
| Ketotifen | 0.87 |
| A | 2.64 |
| B | 0.89 (1.2)** |
| C | 1.43 |
| E | 3.11 |
| Astemizole | 1.08 |

* Dose of compound (i.p.) providing 50% protection against death induced by compound 48/80. Compound was administered 2 hrs. prior to test.

** $ED_{50}$ by p.o. route.

Compound B (example 2) had an approximate $LD_{50}$ in rats of 210 mg/Kg (i.p.) and greater than 500 mg/Kg (p.o.).

Example 18
Formulations

(A)-Injection

| Ingredient | Amount per ampoule |
|---|---|
| Compound of formula (I) | 1.0 mg |
| Water for Injections, q.s. | 1.0 ml |

The finely ground active compound was dissolved in the water for injections. The solution was filtered and sterilized by autoclaving.

(B)-Suppository

| Ingredient | Amount per suppository |
|---|---|
| Compound of Formula (1) | 1.0 mg |
| Cocoa Butter,<br>or Wecobee™ Base q.s. | 2.0 g |

Wecobee is a trademark and is a hydrogenated fatty carboxylic acid.

The finely ground active compound was mixed with the melted suppository base (either Cocoa Butter or Wecobee™ base), poured into moulds and allowed to cool to afford the desired suppositories.

(C)-Syrup

| Ingredient | Amount Per ml |
|---|---|
| Compound of Formula (I) | 1.0 mg |
| Ethanol | 0.3 mg |
| Sucrose | 2.0 mg |
| Methylparaben | 0.5 mg |
| Sodium Benzoate | 0.5 mg |
| Cherry Flavour | q.s. |
| Colouring | q.s. |
| Water | q.s. to 5.0 ml |

Ethanol, sucrose, sodium benzoate, methylparaben, and flavouring were combined in 70% of the total batch quantity of water. Colouring and the active compound were dissolved in the remaining water, then the two solutions were mixed and clarified by filtration.

(D)-Tablet

| Ingredient | Amount per Tablet |
|---|---|
| Compound of Formula (I) | 1.0 mg |
| Lactose | 110.0 mg |
| Corn Starch, Pregelatinized | 2.5 mg |
| Potato Starch | 12.0 mg |
| Magnesium stearate | 0.5 mg |

The active compound was finely ground and intimately mixed with the powdered excipients lactose, corn starch, potato starch and magnesium stearate. The formulation was then compressed to afford a tablet weighing 126 mg.

(E)-Capsule

| Ingredient | Amount per Capsule |
|---|---|
| Compound of Formula (I) | 1.0 mg |
| Lactose | 440.0 mg |
| Magnesium Stearate | 5.0 mg |

The finely ground active compound was mixed with the powdered excipients lactose, corn starch and stearic acid and packed into gelatin capsules.

(F)-Tablet

| Ingredient | Amount per Tablet |
|---|---|
| Compound of Formula (I) | 1.0 mg |
| Pseudoephedrine HCl | 60.0 mg |
| Lactose | 62.5 mg |
| Potato Starch | 14.0 mg |
| Magnesium Stearate | 1.0 mg |
| Gelatin | 2.8 mg |

A tablet was prepared from the above formulation by the method previously described in Example 11 (D).

(G)-Syrup

| Ingredient | Amount per 5 ml |
|---|---|
| Compound of Formula (I) | 1.0 mg |
| Pseudoephedrine HCl | 30.0 mg |
| Codeine Phosphate | 10.0 mg |
| Guaifenesin | 100 mg |
| Methylparaben | 0.5 mg |
| Sodium benzoate | 0.5 mg |
| Flavour | q.s. |
| Colour | q.s. |
| Glycerol | 500 mg |
| Sucrose | 2000 mg |
| Purified Water | q.s. to 5.0 ml |

A syrup containing other active ingredients in addition to a compound of formula (I) was prepared from the above ingredients by an analogous method to that described in Example 11 (C) above.

(H)-Nasal Spray

| Ingredient | Amount per 100.0 ml |
|---|---|
| Compound of Formula (I) | 1 g |
| Sodium Chloride | 0.8 g |
| Preservative | 0.5 g |
| Purified Water | q.s. 100.0 ml |

The preservative was dissolved in warm purified water and after cooling to 25°—30°C the sodium chloride and the compound of formula (I) were added. The pH was then adjusted to 5.5—6.5 and purified water was added to bring the final volume to 100.0 ml.

(I)-Ophthalmic Solution

| Ingredient | Amount per 100.0 ml |
|---|---|
| Compound of Formula (I) | 0.1 g |
| Sodium Chloride | 0.8 g |
| Preservative | 0.5 g |
| Water for Injection | q.s. 100.0 ml |

This formulation was prepared in a similar way to the nasal spray.

(J)-Topical Cream

| Ingredient | Amount per 100.0 ml |
|---|---|
| Compound of Formula (I) | 0.1 g |
| Emulsifying Wax, N.F. | 15.0 g |
| Mineral Oil | 5.0 g |

Scheme 1

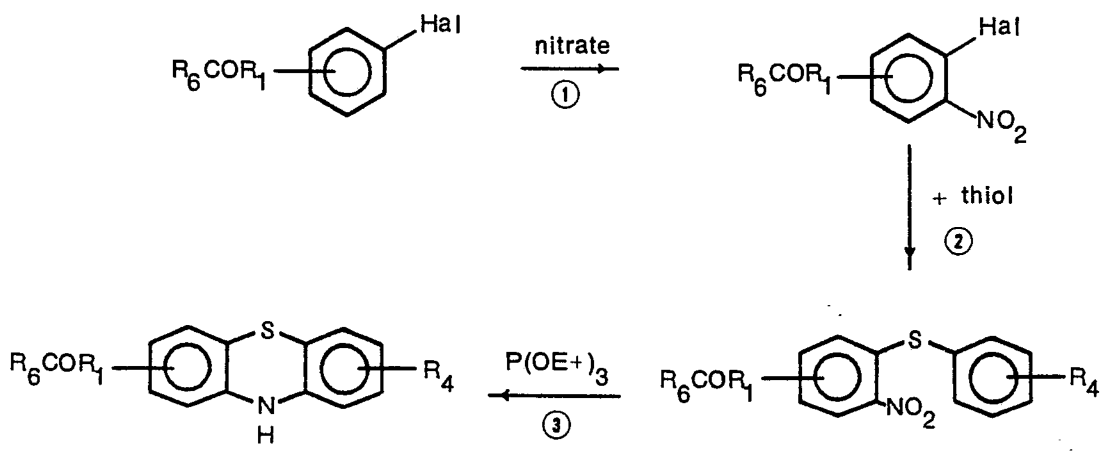


1. nitrating agent, e.g. $HNO_3/H_2SO_4$ at temperature of 0°—100°C conveniently 60°C.

2. Thiol 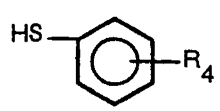

and base, e.g. sodium carbonate in a polar solvent such as an alcohol, e.g. methanol or ethanol at an elevated temperature, e.g. 50°—100°C conveniently at reflux.

3. 2 moles of $P(OEt)_3$ in a high boiling hydrocarbon, for example, n-propylbenzene at an elevated temperature, e.g. 125°—200°C conveiently at reflux.

**Claims for the Contracting States: BE DE FR CH GB IT LI LU NL SE**

1. A compound of the formula (I)

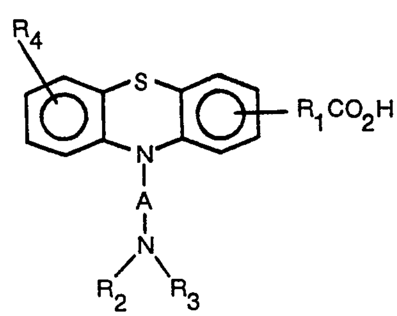

(I)

or a salt thereof; wherein

$R_1$ is a $C_{1-7}$ bivalent aliphatic hydrocarbon group or a single bond;

$R_2$ and $R_3$ are the same or different and are each hydrogen, $C_{1-4}$ alkyl or taken together with the nitrogen comprise a pyrrolidine, piperidine or morpholine ring;

$R_4$ which is attached at the 7- or 8-position is hydrogen, halogen, $C_{1-4}$ alkoxy, $C_{1-4}$ alkyl optionally substituted by one to three halogen atoms; or a group $R_1$ $CO_2H$ as hereinbefore defined; and

A is $C_{1-4}$ alkylene;

other than the compound of formula (1) wherein $R_1CO_2H$ is $-CH_2-CO_2H$ attached at the 2-position of the phenothiazine ring, $R_4$ is hydrogen and $-ANR_2R_3$ is diethylamino ethyl.

2. A compound according to claim 1 of the formula (II):

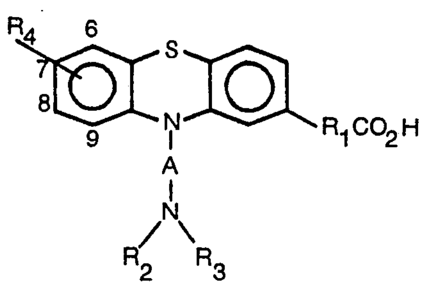

(II)

or a salt thereof; wherein $R_1$ to $R_4$ and A are as defined in relation to the formula (I).

3. A compound according to either claim 1 or 2 wherein $R_1$ is a group $-(CH_2)_n-$ wherein n is an integer 0 to 3, or a group $-CH=CH-$ or a group $-CH(CH_3)(CH_2)_m-$ where m is 0 or 1 or a group $>C(CH_3)_2$.

4. A compound according to any one of claims 1 to 3 wherein $-NR_2R_3$ is a dimethylamino or diethylamino group.

5. A compound according to any one of claims 1 to 4 wherein $R_4$ is hydrogen, methyl, chloro or fluoro.

6. A compound according to any one of claims 1 to 5 wherein A is an ethylene or n- or iso-propylene group.

7. A compound of the formula (I) selected from:

trans-β-[10-2(2-dimethylaminopropyl)phenothiazin-2-yl]acrylic acid
[10-(2-dimethylaminopropyl)phenothiazin-2-yl]acetic acid
[10-(2-dimethylaminopropyl)phenothiazin-2-yl]propionic acid
10-(2-Dimethylaminopropyl)phenothiazine-3-carboxylic acid
*E*-3-(2-Carboxyvinyl)-10-(2-dimethylaminopropyl)phenothiazine
3-(2-Carboxyethyl)-10-(2-dimethylaminopropyl)phenothiazine
10-(2-Dimethylaminopropyl)phenothiazine-4(or 1)-carboxylic acid
(*E*)-4(or 1)-(2-Carboxyvinyl)-10-(2-dimethylaminopropyl)phenothiazine
4-(or 1)-(2-Carboxyethyl)-10-(2-dimethylaminopropyl)phenothiazine
[7-Chloro-10-(2-dimethylaminopropyl)-2-phenothiazinyl]acetic acid
[10-2-Dimethylaminoethyl)-7-methyl-2-phenothiazinyl]acetic acid
[10-(2-Dimethylaminoethyl)-7-fluoro-2-phenothiazinyl]acetic acid
2-[10-(2-Dimethylaminopropyl)-2-phenothiazinyl]-2-methylpropionic acid
2-[10-(3-dimethylaminopropyl)-2-phenothiazinyl]-2-methylpropionic acid
2-Methyl-2-[10-[3-(N-pyrrolidino)propyl]-2-phenothiazinyl]propionic acid
10-(2-Dimethylaminopropyl)phenothiazine-2-carboxylic acid

or a salt thereof.

8. A salt of a compound of the formula (I) according to any one of claims 1 to 7.

9. A compound of the formula (I) or a salt thereof according to claim 1 for use in medicine.

10. A pharmaceutical composition comprising a compound of the formula (I) according to claim 1 together with one or more pharmaceutically carriers thereof.

11. A pharmaceutical composition according to claim 10 which contains other therapeutic ingredients.

12. An analogy method for preparing compounds of the formula (I) according to claim 1, which comprises:

a) The reaction of a compound of the formula (III)

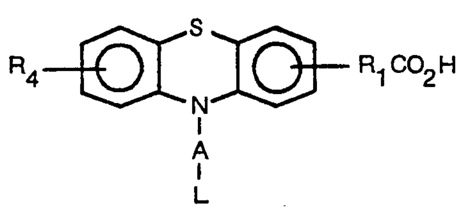

(III)

or an ester thereof with an amine $HNR_2R_3$ wherein A and $R_1$ to $R_4$ are as hereinbefore defined and L is a leaving group;

b) When it is required to prepare a compound of the formula (I) wherein $R_1$ is $(CH_2)_0$ (i) the reaction of a compound of the formula (IV):

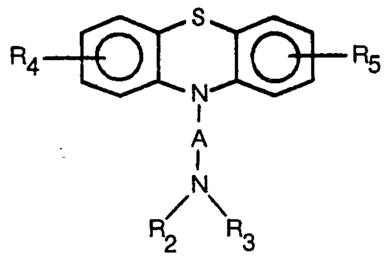

(IV)

wherein $R_2$, $R_3$, $R_4$ and A are as hereinbefore defined and $R_5$ is a hydrogen or halogen atom, with a $C_{1-6}$ alkyl lithium compound followed by treatment with carbon dioxide;

(ii) the oxidation of a compound of the formula (V)

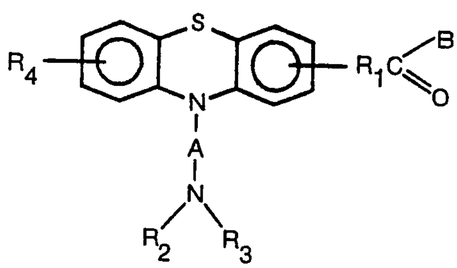

(V)

wherein $R_2$, $R_3$, $R_4$ and A are as hereinbefore defined, $R_1$ is a single bond and B is hydrogen;

c) When it is required to prepare a compound of the formula (I) wherein $R_1$ is $(CH_2)_a$ $CH{=}CH(CH_2)_b$) and a is 0 and b is 0 to 5 the reaction of a compound of the formula (V) as hereinbefore defined, with a Wittig reagent suitable for attaching the side chain $CH{=}CH(CH_2)_b$ $COR_6$ wherein $COR_6$ is an acid, ester or amide group followed if necessary by deprotection of the carboxy group;

d) When it is required to prepare a compound of the formula (I) wherein $R_1$ is CH=CH, the reaction of a compound of the formula (V) as hereinbefore defined, with malonic acid or a malonic acid ester;

e) the hydrolysis of a compound of the formula (IV):

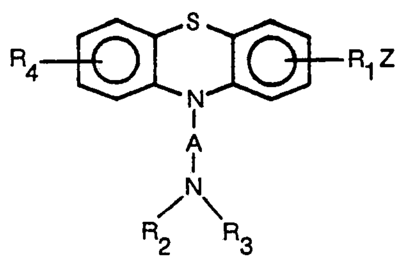

(VI)

wherein $R_2$ to $R_4$ and A are as hereinbefore defined and $R_1$ Z is a group $R_1$ CN wherein $R_1$ is a $C_{1-7}$ bivalent hydrocarbon group, or a group $R_7$ — CH=C(Y)XH wherein $R_7$ is a $C_{1-6}$ bivalent hydrocarbon group or a single bond, X is oxygen or sulphur and Y is an amino group;

f) the alkylation of a compound of the formula (VII):

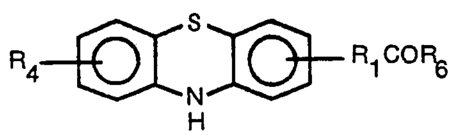

(VII)

wherein $R_1$ and $R_4$ are as hereinbefore defined, $COR_6$ is an ester or amide group, followed by deprotection of the carboxy group; and thereafter, optionally converting one compound of the formula (I) to another compound of the formula (I) by methods well known to those skilled in the art, for example the reduction of one or more double bonds.

**Claims for the Contracting State: AT**

1. An analogy method for preparing compounds of the formula (I)

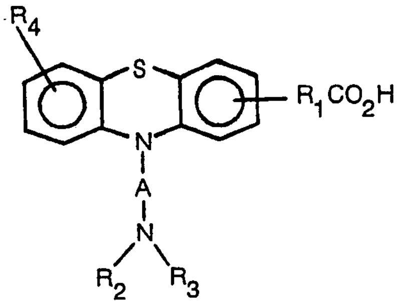

(I)

or a salt thereof; wherein

$R_1$ is a $C_{1-7}$ bivalent aliphatic hydrocarbon group or a single bond;

$R_2$ and $R_3$ are the same or different and are each hydrogen, $C_{1-4}$ alkyl or taken together with the nitrogen comprise a pyrrolidine, piperidine or morpholine ring;

$R_4$ which is attached at the 7- or 8-position is hydrogen, halogen, $C_{1-4}$ alkoxy, $C_{1-4}$ alkyl optionally substituted by one to three halogen atoms; or a group $R_1$ $CO_2H$ as hereinbefore defined; and

A is $C_{1-4}$ alkylene;

other than the compound of formula (1) wherein $R_1CO_2H$ is $—CH_2—CO_2H$ attached at the 2-position of the phenothiazine ring, $R_4$ is hydrogen and $—ANR_2R_3$ is diethylamino ethyl,

which comprises:

a) the reaction of a compound of the formula (III)

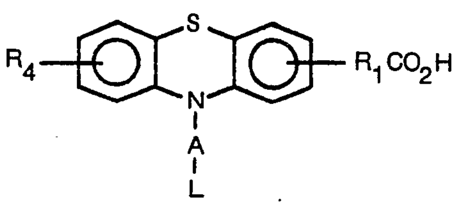

(III)

or an ester thereof with an amine $HNR_2R_3$ wherein A and $R_1$ to $R_4$ are as hereinbefore defined and L is a leaving group;

b) when it is required to prepare a compound of the formula (I) wherein $R_1$ is $(CH_2)_0$ (i) the reaction of a compound of the formula (IV):

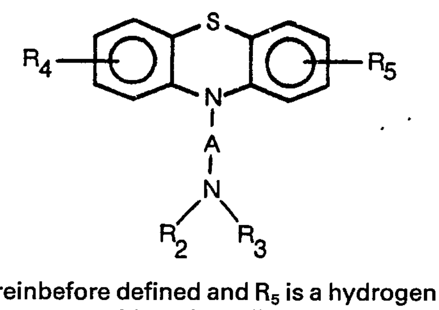

(IV)

wherein $R_2$, $R_3$, $R_4$ and A are as hereinbefore defined and $R_5$ is a hydrogen or halogen atom, with a $C_{1-6}$ alkyl lithium compound followed by treatment with carbon dioxide;

(ii) the oxidation of a compound of the formula (V)

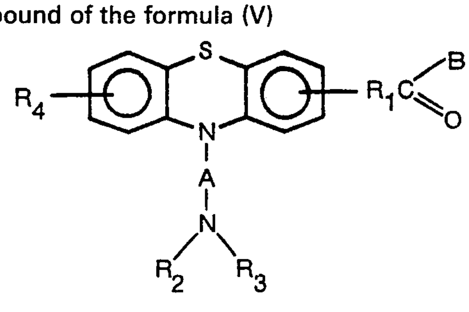

(V)

wherein $R_2$, $R_3$, $R_4$ and A are as hereinbefore defined, $R_1$ is a single bond and B is hydrogen;

c) when it is required to prepare a compound of the formula (I) wherein $R_1$ is $(CH_2)_a CH{=}CH(CH_2)_b$ and a is 0 and b is 0 to 5 the reaction of a compound of the formula (V) as hereinbefore defined, with a Wittig reagent suitable for attaching the side chain $CH{=}CH(CH_2)_b COR_6$ wherein $COR_6$ is an acid, ester or amide group followed if necessary by deprotection of the carboxy group;

d) when it is required to prepare a compound of the formula (I) wherein $R_1$ is CH=CH, the reaction of a compound of the formula (V) as hereinbefore defined, with malonic acid or a malonic acid ester;

e) the hydrolysis of a compound of the formula (IV):

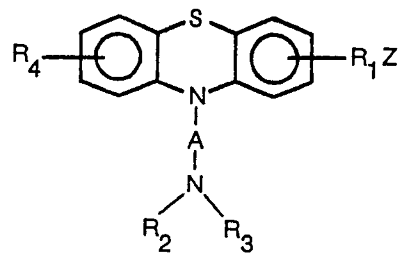

(VI)

wherein $R_2$ to $R_4$ and A are as hereinbefore defined and $R_1$ Z is a group $R_1$ CN wherein $R_1$ is a $C_{1-7}$ bivalent hydrocarbon group, or a group $R_7$—CH=C(Y)XH wherein $R_7$ is a $C_{1-6}$ bivalent hydrocarbon group or a single bond, X is oxygen or sulphur and Y is an amino group;

f) the alkylation of a compound of the formula (VII):

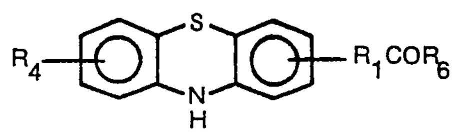

(VII)

wherein $R_1$ and $R_4$ are as hereinbefore defined, $COR_6$ is an ester or amide group, followed by deprotection of the carboxy group; and thereafter, optionally converting one compound of the formula (I) to another compound of the formula (I) by methods well known to those skilled in the art, for example the reduction of one or more double bonds.

2. A method according to claim 1 for the preparation of a compound of the formula (II):

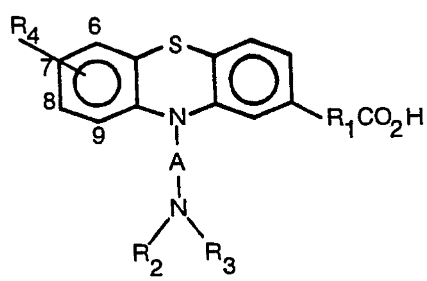

(II)

or a salt thereof; wherein $R_1$ to $R_4$ and A are as defined in relation to formula (I).

3. A method according to claim 1 for the preparation of a compound according to either claim 1 or 2 wherein $R_1$ is a group $-(CH_2)_n-$ wherein n is an integer 0 to 3, or a group —CH=CH— or a group $-CH(CH_3)(CH_2)_m-$ where m is 0 or 1 or a group $>C(CH_3)_2$.

4. A method according to claim 1 for the preparation of a compound according to any one of the claims 1 to 3 wherein $-NR_2R_3$ is a dimethylamino or diethylamino group.

5. A method according to claim 1 for the preparation of a compound according to anyone of claims 1 to 4 wherein $R_4$ is hydrogen, methyl, chloro or fluoro.

6. A method according to claim 1 for the preparation of a compound according to any one of claims 1 to 5 wherein A is an ethylene or n- or iso-propylene group.

7. A method according to claim 1 for the preparation of a compound of the formula (I) selected from:
trans-β-[10-2(2-dimethylaminopropyl)phenothiazin-2-yl]acrylic acid
2-[10-(2-dimethylaminopropyl)phenothiazin-2-yl]acetic acid
2-[10-(2-dimethylaminopropyl)phenothiazin-2-yl]propionic acid
10-(2-Dimethylaminopropyl)phenothiazine-3-carboxylic acid
*E*-3-(2-Carboxyvinyl)-10-(2-dimethylaminopropyl)phenothiazine
3-(2-Carboxyethyl)-10-(2-dimethylaminopropyl)phenothiazine
10-(2-Dimethylaminopropyl)phenothiazine-4(or 1)-carboxylic acid
(*E*)-4(or 1)-(2-Carboxyvinyl)-10-(2-dimethylaminopropyl)phenothiazine

4-(or 1)-(2-Carboxyethyl)-10-(2-dimethylaminopropyl)phenothiazine
[7-Chloro-10-(2-dimethylaminopropyl)-2-phenothiazinyl]acetic acid
[10-(2-Dimethylaminoethyl)-7-methyl-2-phenothiazinyl]acetic acid
[10-(2-Dimethylaminoethyl)-7-fluoro-2-phenothiazinyl]acetic acid
2-[10-(2-Dimethylaminopropyl)-2-phenothiazinyl]-2-methylpropionic acid
2-[10-(3-dimethylaminopropyl)-2-phenothiazinyl]-2-methylpropionic acid
2-Methyl-2-[10-[3-(N-pyrrolidino)propyl]-2-phenothiazinyl]propionic acid
10-(2-Dimethylaminopropyl)phenothiazine-2-carboxylic acid
or salts thereof.

8. A method for preparing a pharmaceutical composition which comprises bringing a compound of the formula (I), according to claim 1, into admixture with one or more pharmaceutically acceptable carriers therefor.

9. A method according to claim 8 wherein other therapeutic ingredients are brought into admixture with the compound of the formula (I) and the pharmaceutically acceptable barriers.

**Patentansprüche für die Vertragsstaaten: BE DE FR CH GB IT LI LU NL SE**

1. Verbindung der Formel (I)

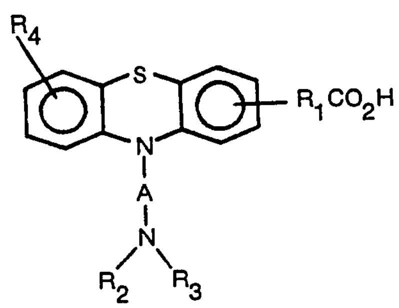

(I)

oder ein Salz hievon, worin

$R_1$ eine zweiwertige aliphatische $C_{1-7}$-Kohlenwasserstoffgruppe oder eine Einfachbindung bedeutet;

$R_2$ und $R_3$ gleich oder verschieden sind und jeweils Wasserstoff oder $C_{1-4}$-Alkyl darstellen oder zusammen mit dem Stickstoffatom einen Pyrrolidin-, Piperidin- oder Morpholinring bilden;

$R_4$, das sich in Stellung 7 oder 8 befindet, Wasserstoff, Halogen, $C_{1-4}$-Alkoxy, $C_{1-4}$-Alkyl gegebenenfalls substituiert durch ein bis drei Halogenatome oder eine Gruppe $R_1CO_2H$, wie oben definiert, ist; und

A $C_{1-4}$-Alkylen darstellt;

die sich von der Verbindung der Formel (I) unterscheidet, worin $R_1CO_2H$ in Stellung 2 des Phenothiazinringes befindliches $—CH_2—CO_2H$, $R_4$ Wasserstoff und $ANR_2R_3$ Diäthylaminöathyl bedeuten.

2. Verbindung nach Anspruch 1 der Formel (II)

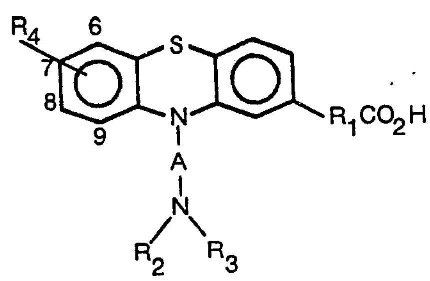

(II)

oder ein Salz hievon, worin $R_1$ bis $R_4$ und A wie in bezug auf Formel (I) definiert sind.

3. Verbindung nach Anspruch 1 oder 2, woring $R_1$ eine Gruppe $—(CH_2)_n—$, wobei n eine ganze Zahl von Null bis 3 ist, eine Gruppe $—CH=CH—$, eine Gruppe $—CH(CH_3)(CH_2)_m—$, wobei m Null oder 1 ist, oder eine Gruppe $>C(CH_3)_2$ bedeutet.

4. Verbindung nach einem der Ansprüche 1 bis 3, worin $NR_2R_3$ eine Dimethylamino- oder Diäthylaminogruppe ist.

5. Verbindung nach einem der Ansprüche 1 bis 4, worin $R_4$ Wasserstoff, Methyl, Chlor oder Fluor ist.

6. Verbindung nach einem der Ansprüche 1 bis 5, worin A eine Äthylen- oder n- oder Isopropylengruppe ist.

7. Verbindung der Formel (I) ausgewählt aus

trans-β-[10-(2-Dimethylaminopropyl)-phenothiazin-2-yl]-acrylsäure,

[10-(2-Dimethylaminopropyl)-phenothiazin-2-yl]-essigsäure,
[10-(2-Dimethylaminopropyl)-phenothiazin-2-yl]-propionsäure,
10-(2-Dimethylaminopropyl)-phenothiazin-3-carbonsäure,
*E*-3-(2-Carboxyvinyl)-10-(2-dimethylaminopropyl)-phenothiazin,
3-(2-Carboxyäthyl)-10-(2-dimethylaminopropyl)-phenothiazin,
10-(2-Dimethylaminopropyl)-phenothiazin-4(oder 1)-carbonsäure,
(*E*)-4(oder 1)-(2-Carboxyvinyl)-10-(2-dimethylaminopropyl)-phenothiazin,
4-(oder 1)-(2-Carboxyäthyl)-10-(2-dimethylaminopropyl)-phenothiazin,
[7-Chloro-10-(2-dimethylaminopropyl)-2-phenothiazinyl]-essigsäure,
[10-(2-Dimethylaminoäthyl)-7-methyl-2-phenothiazinyl]-essigsäure,
[10-(2-Dimethylaminoäthyl)-7-fluor-2-phenothiazinyl]-essigsäure,
2-[10-(2-Dimethylaminopropyl)-2-phenothiazinyl]-2-methylpropionsäure,
2-[10-(3-Dimethylaminopropyl)-2-phenothiazinyl]-2-methylpropionsäure,
2-Methyl-2-[10-[3-(N-pyrrolidino)propyl]-2-phenothiazinyl]-propionsäure,
10-(2-Dimethylaminopropyl)phenothiazin-2-carbonsäure
oder ein Salz hievon.

8. Ein Salz einer Verbindung der Formel (I) nach einem der Ansprüche 1 bis 7.

9. Verbindung der Formel (I) oder ein Salz hievon nach Anspruch 1 zur Verwendung in der Medizin.

10. Pharmazeutische Zusammensetzung umfassend eine Verbindung der Formel (I) nach Anspruch 1 zusammen mit einem oder mehreren pharmazeutisch annehmbaren Trägern hiefür.

11. Pharmazeutische Zusammensetzung nach Anspruch 10, die andere therapeutische Bestandteile enthält.

12. Analogieverfahren zum Herstellen von Verbindungen der Formel (I) nach Anspruch 1, welches umfaßt:

a) die Umsetzung einer Verbindung der Formel (III)

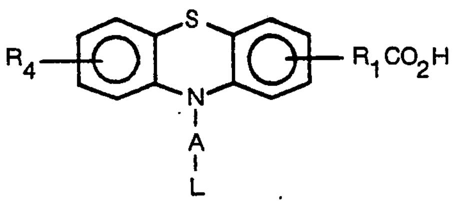

(III)

oder eines Esters hievon mit einem Amin $HNR_2R_3$, worin A und $R_1$ bis $R_4$ wie oben definiert sind und L eine abspaltbare Gruppe darstellt;

b) wenn eine Verbindung der Formel (I) hergestellt werden soll, worin $R_1$ die Bedeutung $(CH_2)_0$ hat,

(i) die Umsetzung einer Verbindung der Formel (IV)

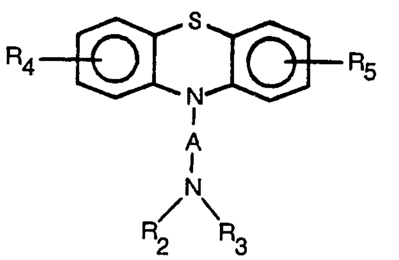

(IV)

worin $R_2$, $R_3$, $R_4$ und A wie oben definiert sind und $R_5$ ein Wasserstoff- oder Halogenatom darstellt, mit einer $C_{1-6}$-Alkyllithium- verbindung und nachfolgende Behandlung mit Kohlendioxid:

(ii) die Oxidation einer Verbindung der Formel (V)

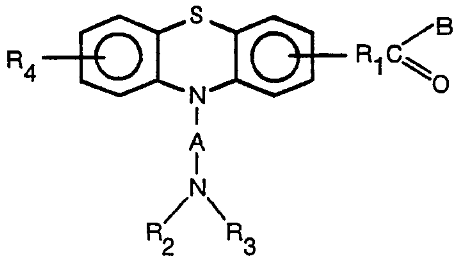

(V)

worin $R_2$, $R_3$, $R_4$ und A wie oben definiert sind, $R_1$ eine Einfachbindung darstellt und B Wasserstoff ist;

c) wenn eine Verbindung der Formel (I) hergestellt werden soll, worin $R_1$ die Bedeutung

$(CH_2)_aCH{=}CH(CH_2)_b$ hat, wobei a Null ist und b Null bis 5 ist, die Umsetzung einer Verbindung der Formel (V), wie oben definiert, mit einem Wittig-Reagens, das geeignet ist, die Seitenkette $CH{=}CH(CH_2)_bCOR_6$ anzufügen, worin $COR_6$ eine Säure-, Ester- oder Amidgruppe ist, gefolgt, wenn notwendig, von der Entfernung der Schutzgruppe der Carboxygruppe;

d) wenn eine Verbindung der Formel (I) hergestellt werden soll, worin R, die Bedeutung CH=CH hat, die Umsetzung einer Verbindung der Formel (V), wie oben definiert, mit Malonsäure oder einem Malonsäureester;

e) die Hydrolyse einer Verbindung der Formel (VI)

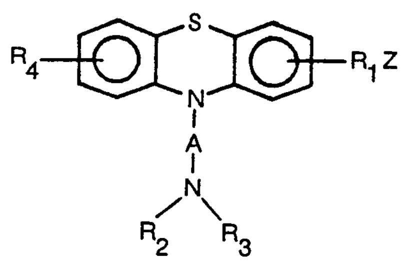

(VI)

worin $R_2$ bis $R_4$ und A wie oben definiert sind und $R_1Z$ eine Gruppe $R_1CN$, wobei $R_1$ eine Zweiwertige $C_{1-7}$-Kohlenwasserstoffgruppe ist, oder eine Gruppe $R_7$—$CH{=}C(Y)XH$, wobei $R_7$ eine zweiwertige $C_{1-6}$-Kohlenwasserstoffgruppe oder eine Einfachbindung, X Sauerstoff oder Schwefel und Y eine Aminogruppe sind, bedeutet;

f) die Alkylierung einer Verbindung der Formel (VII)

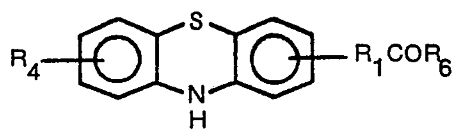

(VII)

worin $R_1$ und $R_4$ wie oben definiert sind und $COR_6$ eine Ester- oder Amidgruppe bedeutet, gefolgt von der Entfernung der Schutzgruppe der Carboxygruppe und

gegebenenfalls das anschließende Überführen einer Verbindung der Formel (I) durch den Fachleuten wohlbekannte Verfahren, beispielsweise Reduktion einer oder mehrerer Doppelbindungen, in eine andere Verbindung der Formel (I).

**Patentansprüche für den Vertragsstaat: AT**

1. Analogieverfahren zum Herstellen von Verbindungen der Formel (I)

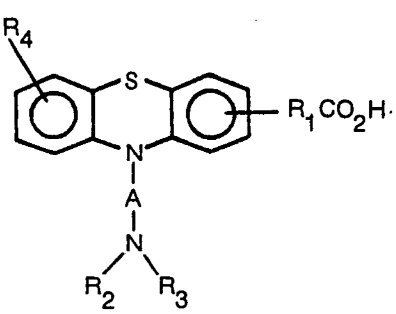

(I)

oder ein Salz hievon, worin

$R_1$ eine zweiwertige aliphatische $C_{1-7}$-Kohlenwasserstoffgruppe oder eine Einfachbindung bedeutet;

$R_2$ und $R_3$ gleich oder verschieden sind und jeweils Wasserstoff oder $C_{1-4}$-Alkyl darstellen oder zusammen mit dem Stickstoffatom einen Pyrrolidin-, Piperidin- oder Morpholinring bilden;

$R_4$, das sich in Stellung 7 oder 8 befindet, Wasserstoff, Halogen, $C_{1-4}$-Alkoxy, $C_{1-4}$-Alkyl gegebenenfalls substituiert durch ein bis drei Halogenatome oder eine Gruppe $R_1CO_2H$, wie oben definiert, ist; und

A $C_{1-4}$-Alkylen darstellt;

die sich von der Verbindung der Formel (I) unterscheidet, worin $R_1CO_2H$ in Stellung 2 des Phenothiazinringes befindliches —$CH_2$—$CO_2H$, $R_4$ Wasserstoff und $ANR_2R_3$ Diäthylaminöathyl bedeuten, welches umfaßt:

a) die Umsetzung einer Verbindung der Formel (III)

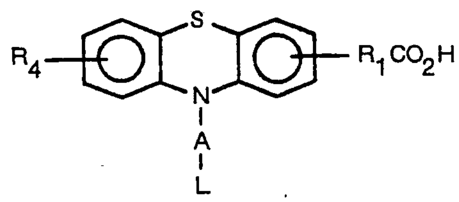

(III)

oder eines Esters hievon mit einem Amin $HNR_2R_3$, worin A und $R_1$ bis $R_4$ wie oben definiert sind und L eine abspaltbare Gruppe darstellt;

b) wenn eine Verbindung der Formel (I) hergestellt werden soll, worin $R_1$ die Bedeutung $(CH_2)_0$ hat,

(i) die Umsetzung einer Verbindung der Formel (IV)

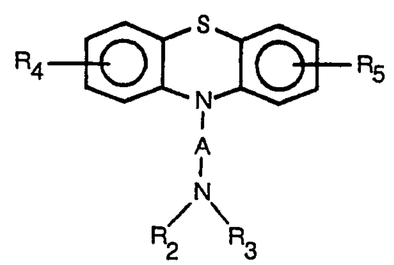

(IV)

worin $R_2$, $R_2$, $R_4$ und A wie oben definiert sind und $R_5$ ein Wasserstoff- oder Halogenatom darstellt, mit einer $C_{1-6}$-Alkyllithiumverbindung und nachfolgende Behandlung mit Kohlendioxid;

(ii) die Oxidation einer Verbindung der Formel (V)

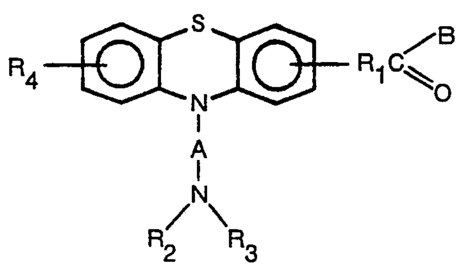

(V)

worin $R_2$, $R_3$, $R_4$ und A wie oben definiert sind, $R_1$ einme Einfachbindung darstellt und B Wasserstoff ist;

c) wenn eine Verbindung der Formel (I) hergestellt werden soll, worin $R_1$ die Bedeutung $(CH_2)_aCH=CH(CH_2)_b$ hat, wobei a Null ist und b Null bis 5 ist, die Umsetzung einer Verbindung der Formel (V), wie oben definiert, mit einem Wittig-Reagens, das geeignet ist, die Seitenkette $CH=CH(CH_2)_bCOR_6$ anzufügen, worin $COR_6$ eine Säure-, Ester- oder Amidgruppe ist, gefolgt, wenn notwendig, von der Entfernung der Schutzgruppe der Carboxygruppe;

d) wenn eine Verbindung der Formel (I) hergestellt werden soll, worin $R_1$ die Bedeutung CH=CH hat, die Umsetzung einer Verbindung der Formel (V), wie oben definiert, mit Malonsäure oder einem Malonsäureester;

e) die Hydrolyse einer Verbindung der Formel (VI)

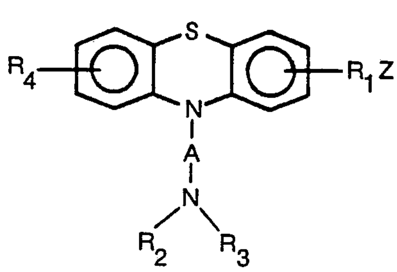

(VI)

worin $R_2$ bis $R_4$ und A wie oben definiert sind und $R_1Z$ eine Gruppe $R_1CN$, wobei $R_1$ eine zweiwertige $C_{1-7}$-Kohlenwasserstoffgruppe ist, oder eine Gruppe $R_7$—CH=C(Y)XH, wobei $R_7$ eine zweiwertige $C_{1-6}$-Kohlenwasserstoffgruppe oder eine Einfachbindung, X Sauerstoff oder Schwefel und Y eine Aminogruppe sind, bedeutet;

f) die Alkylierung einer Verbindung der Formel (VII)

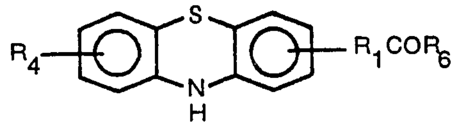

(VII)

worin $R_1$ und $R_4$ wie oben definiert sind und $COR_6$ eine Ester- oder Amidgruppe bedeutet, gefolgt von der Entfernung der Schutzgruppe der Carboxygruppe und

gegebenenfalls das anschließende Überführen einer Verbindung der Formel (I) durch den Fachleuten wohlbekannte Verfahren, beispielsweise Reduktion einer oder mehrerer Doppelbindungen, in eine andere Verbindung der Formel (1).

2. Verfahren nach Anspruch 1 zur Herstellung einer Verbindung der Formel (II)

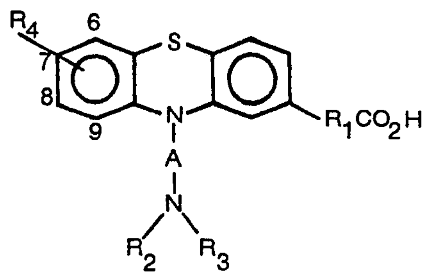

(II)

oder eines Salzes hievon, worin $R_1$ bis $R_4$ und A wie in bezug auf Formel (I) definiert sind.

3. Verfahren nach Anspurch 1 zur Herstellung einer Verbindung nach Anspruch 1 oder 2, worin $R_1$ eine Gruppe $-(CH_2)_n-$, wobei n eine ganze Zahl von Null bis 3 ist, eine Gruppe $-CH{=}CH-$, eine Gruppe $-CH(CH_3)(CH_2)_m-$, wobei m Null oder 1 ist, oder eine Gruppe $>C(CH_3)_2$ bedeutet.

4. Verfahren nach Anspruch 1 zur Herstellung einer Verbindung nach einem der Ansprüche 1 bis 3, worin $-NR_2R_3$ eine Dimethylamino- oder Diäthylaminogruppe ist.

5. Verfahren nach Anspruch 1 zur Herstellung einer Verbindung nach einem der Ansprüche 1 bis 4, worin $R_4$ Wasserstoff, Methyl, Chlor oder Fluor ist.

6. Verfahren nach Anspuch 1 zur Herstellung einer Verbindung nach einem der Ansprüche 1 bis 5, worin A eine Äthylen- oder n- oder Isopropylengruppe ist.

7. Verfahren nach Anspruch 1 zur Herstellung einer Verbindung der Formel (I) ausgewählt aus
trans-β-[10-(2-Dimethylaminopropyl)-phenothiazin-2-yl]-acrylsäure,
[10-(2-Dimethylaminopropyl)-phenothiazin-2-yl]-essigsäure,
[10-(2-Dimethylaminopropyl)-phenothiazin-2-yl]-propionsäure,
10-(2-Dimethylaminopropyl)-phenothiazin-3-carbonsäure,
*E*-3-(2-Carboxyvinyl)-10-(2-dimethylaminopropyl)-phenothiazin,
3-(2-Carboxyäthyl)-10-(2-dimethylaminopropyl)-phenothiazin,
10-(2-Dimethylaminopropyl)-phenothiazin-4(oder 1)-carbonsäure,
(*E*)-4(oder 1)-(2-Carboxyvinyl)-10-(2-dimethylaminopropyl)-phenothiazin,
4-(oder 1)-(2-Carboxyäthyl)-10-(2-dimethylaminopropyl)-phenothiazin,
[7-Chloro-10-(2-dimethylaminopropyl)-2-phenothiazinyl]-essigsäure,
[10-2-Dimethylaminoäthyl)-7-methyl-2-phenothiazinyl]-essigsäure,
[10-(2-Dimethylaminoäthyl)-7-fluor-2-phenothiazinyl]-essigsäure,
2-[10-(2-Dimethylaminopropyl)-2-phenothiazinyl]-2-methylpropionsäure,
2-[10-(3-Dimethylaminopropyl)-2-phenothiazinyl]-2-methylpropionsäure,
2-Methyl-2-[10-[3-(N-pyrrolidino)propyl]-2-phenothiazinyl]-propionsäure,
10-(2-Dimethylaminopropyl)phenothiazin-2-carbonsäure
oder ein Salz hievon.

8. Verfahren zum Herstellen einer pharmazeutischen Zusammensetzung, welches das Mischen einer Verbindung der Formel (I) nach Anspruch 1 mit einem oder mehreren pharmazeutisch annehmbaren Trägern hiefür umfaßt.

9. Verfahren nach Anspruch 8, worin andere therapeutische Bestandteile mit der Verbindung der Formel (I) und den pharmazeutisch annehmbaren Trägern gemischt werden.

**Revendications pour les Etats contractants: BE DE FR CH GB IT LI LU NL SE**

1. Composé de formule (I)

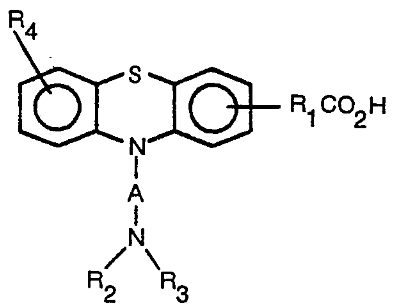

(I)

ou un sel de celui-ci,

où $R_1$ est un radical hydrocarboné aliphatique bivalent en $C_{1-7}$ ou un liaison simple,

$R_2$ et $R_3$ sont identiques ou différentes et sont chacun hydrogène ou $C_{1-4}$-alcoyle ou bien pris ensemble avec l'azote forment un cycle pyrrolidine, pipéridine ou morpholine;

$R_4$ qui est fixé à la position 7 ou 8 est hydrogène, halogène, $C_{1-4}$-alcoxy, $C_{1-4}$-alcoyle éventuellement substitué par un à trois atomes d'halogène; ou un radical $R_1CO_2H$ tel que défini ci-dessus; et

A est $C_{1-4}$-alcoylène;

autre que le composé de formule (I) où $R_1CO_2H$ ets $-CH_2-CO_2H$ fixé à la position 2 du cycle phénothiazine, $R_4$ est hydrogène et $ANR_2R_3$ est diéthylaminoéthyle.

2. Composé suivant la revendication 1, de formule (II)

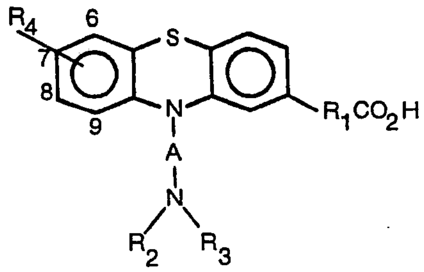

(II)

ou un sel de celui-ci,

où $R_1$ à $R_4$ et A sont tels que définis pour la formule (I).

3. Composé suivant la revendication 1 ou 2, dans lequel $R_1$ est un radical $-(CH_2)_n-$ où n est un entier de 0 à 3, ou un radical $-CH=CH-$, ou un radical $-CH(CH_3)(CH_2)_m-$ où m est 0 ou 1, ou un radical $-C(CH_3)_2-$.

4. Composé suivant l'une quelconque des revendications 1 à 3, dans lequel $NR_2R_3$ est un radical diméthylamino ou diéthylamino.

5. Composé suivant l'une quelconque des revendications 1 à 4, dans lequel $R_4$ est hydrogène, méthyle, chloro ou fluoro.

6. Composé suivant l'une quelconque des revendications 1 à 5, dans lequel A est un radical éthylène ou n- ou iso-propylène.

7. Composé de formule (I) choisi parmi:

l'acide trans-β-[10-2-(2-diméthylaminopropyl)phénothiazin-2-yl]acrylique
l'acide [10-(2-diméthylaminopropyl)phénothiazin-2-yl]acétique
l'acide [10-(2-diméthylaminopropyl)phénothiazin-2-yl]propionique
l'acide 10-(2-diméthylaminopropyl)phénothiazine-3-carboxylique
la *E*-3-(2-carboxyvinyl)-10-(2-diméthylaminopropyl)phénothiazine
la 3-(2-carboxyéthyl)-10-(2-diméthylaminopropyl)phénothiazine
l'acide 10-(2-diméthylaminopropyl)phénothiazine-4(ou 1)-carboxylique
la (*E*)-4(ou 1)-(2-carboxyvinyl)-10-(2-diméthylaminopropyl)phénothiazine
la 4-(ou 1)-(2-carboxyéthyl)-10-(2-diméthylaminopropyl)phénothiazine
l'acide [7-chloro-10-(2-diméthylaminopropyl)-2-phénothiazinyl]acétique
l'acide [10-(2-diméthylaminoéthyl)-7-méthyl-2-phénothiazinyl]acétique
l'acide [10-(2-diméthylaminoéthyl)-7-fluoro-2-phénothiazinyl]acétique
l'acide 2-[10-(2-diméthylaminopropyl)-2-phénothiazinyl]-2-méthylpropionique
l'acide 2-[10-(3-diméthylaminopropyl)-2-phénothiazinyl]-2-méthylpropionique
l'acide 2-méthyl-2-[10-[3-(N-pyrrolidino)propyl]-2-phénothiazinyl]propionique

l'acide 10-(2-diméthylaminopropyl)phénothiazine-2-carboxylique
et leurs sels.

8. Sel d'un composé de formule (I) suivant l'une quelconque des revendications 1 à 7.

9. Composé de formule (I) ou sel de celui-ci suivant la revendication 1, à utiliser en médecine.

10. Composition pharmaceutique comprenant un composé de formule (I) suivant la revendication 1 conjointement avec un ou plusieurs excipients pharmaceutiquement acceptables.

11. Composition pharmaceutique suivant la revendication 10, qui contient d'autres constituants thérapeutiques.

12. Procédé par analogie pour préparer les composés de formule (I) suivant la revendication 1, qui comprend:

a) la réaction d'un composé de formule (III)

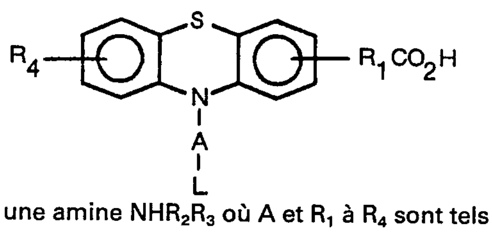

(III)

ou d'un ester de celui-ci, avec une amine $NHR_2R_3$ où A et $R_1$ à $R_4$ sont tels que définis ci-dessus et L est un radical partant;

b) lorsqu'il est requis de préparer un composé de formule (I) où $R_1$ est $(CH_2)_0$

(i) la réaction d'un composé de formule (IV):

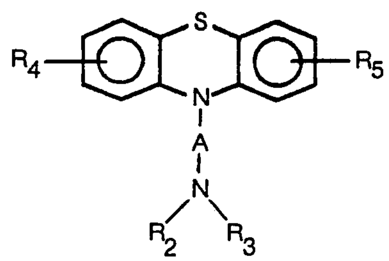

(IV)

où $R_2$, $R_3$, $R_4$ et A sont tels que définis ci-dessus et $R_5$ est un atome d'hydrogène ou d'halogène, avec un $C_{1-6}$-alcoyllithium, suivie du traitement au moyen de dioxyde de carbone;

(ii) l'oxydation d'un composé de formule (V):

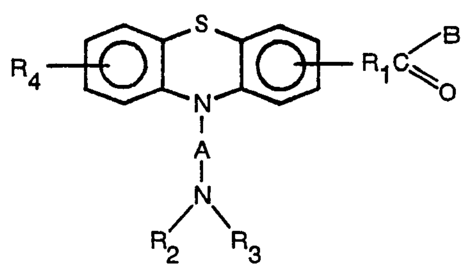

(V)

où $R_2$, $R_3$, $R_4$ et A sont tels que définis ci-dessus, $R_1$ est une liaison simple et B est hydrogène;

c) lorsqu'il est requis de préparer un composé de formule (I) où $R_1$ est $(CH_2)_aCH{=}CH(CH_2)_b$ et a est 0 et b est 0 à 5, la réaction d'un composé de formule (V) tel que défini ci-dessus avec un réactif de Witting propre à fixer la chaîne latérale $CH{=}CH(CH_2)_bCOR_6$, où $COR_6$ est un radical acide, ester ou amide, suivie de la déprotection du radical carboxyle;

d) lorsqu'il est requis de préparer un composé de formule (I) où $R_1$ est $CH{=}CH$, la réaction d'un composé de formule (V) tel que défini ci-dessus avec l'acide malonique ou un ester de l'acide malonique;

e) l'hydrolyse d'un composé de formule (VI):

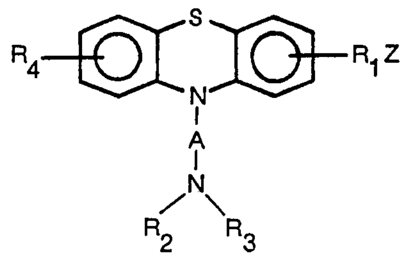

(VI)

où $R_2$ à $R_4$ et A sont tels que définis ci-dessus et $R_1Z$ est un radical $R_1CN$ où $R_1$ est un radical hydrocarboné

bivalent en $C_{1-7}$ ou un radical $R_7$—CH=C(Y)XH où $R_7$ est un radical hydrocarboné bivalent en $C_{1-6}$ ou une liaison simple, X est oxygène ou soufre et Y est un radical amino secondaire;

f) l'alcoylation d'un composé de formule (VII):

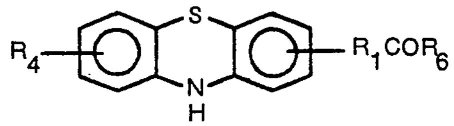

(VII)

où $R_1$ et $R_4$ sont tels que définis ci-dessus et $COR_6$ est un radical ester ou amide, suivie de la déprotection; et ensuite, la conversion éventuelle d'un composé de formule (I) en un autre composé de formule (I) suivant des procédés connus de l'homme de métier, par exemple la réduction d'une ou de plusieurs doubles liaisons.

**Revendications pour l'Etat contractant: AT**

1. Procédé par analogie pour préparer les composés de formule (I)

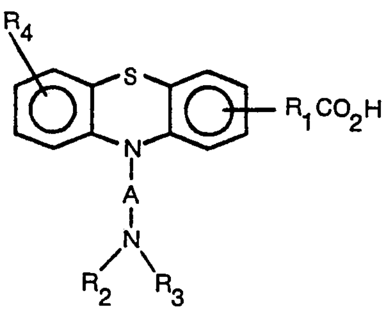

(I)

ou un sel de ceux-ci,

où $R_1$ est un radical hydrocarboné aliphatique bivalent en $C_{1-7}$ ou un liaison simple,

$R_2$ et $R_3$ sont identiques ou différentes et sont chacun hydrogène ou $C_{1-4}$-alcoyle ou bien pris ensemble avec l'azote forment un cycle pyrrolidine, pipéridine ou morpholine;

$R_4$ qui est fixé à la position 7 ou 8 est hydrogène, halogène, $C_{1-4}$-alcoxy, $C_{1-4}$-alcoyle éventuellement substitué par un à trois atomes d'halogène; ou un radical $R_1CO_2H$ tel que défini ci-dessus; et

A est $C_{1-4}$-alcoylène;

autre que le composé de formule (I) où $R_1CO_2H$ est —$CH_2$—$CO_2H$ fixé à la position 2 du cycle phénothiazine, $R_4$ est hydrogène et $ANR_2R_3$ est diéthylaminoéthyle,

qui comprend:

a) la réaction d'un composé de formule (III)

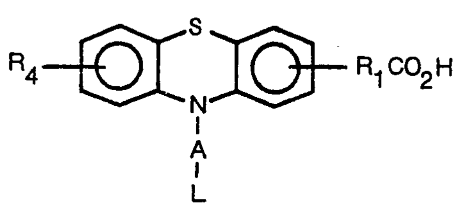

(III)

ou d'un ester de celui-ci, avec une amine $NHR_2R_3$ où A et $R_1$ à $R_4$ sont tels que définis ci-dessus et L est un radical partant;

b) lorsqu'il est requis de préparer un composé de formule (I) où $R_1$ est $(CH_2)_0$

(i) la réaction d'un composé de formule (IV):

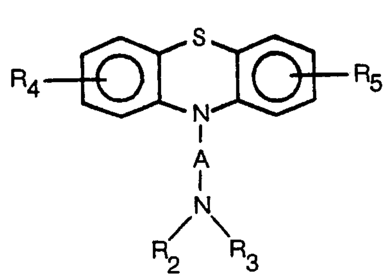

(IV)

où $R_2$, $R_3$, $R_4$ et A sont tels que définis ci-dessus et $R_5$ est un atome d'hydrogène ou d'halogène, avec un $C_{1-6}$-alcoyllithium, suivie du traitement au moyen de dioxyde de carbone;

(ii) l'oxydation d'un composé de formule (V):

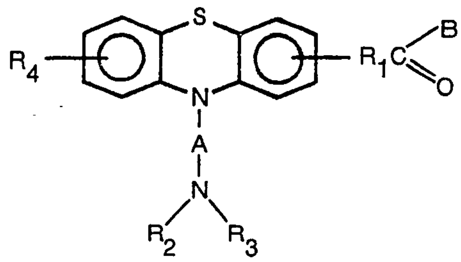

(V)

où $R_2$, $R_3$, $R_4$ et A sont tels que définis ci-dessus, $R_1$ est une liaison simple et B est hydrogène;

c) lorsqu'il est requis de préparer un composé de formule (I) où $R_1$ est $(CH_2)_aCH{=}CH(CH_2)_b$ et a est 0 et b est 0 à 5, la réaction d'un composé de formule (V) tel que défini ci-dessus avec un réactif de Witting propre à fixer la chaîne latérale $CH{=}CH(CH_2)_bCOR_6$, où $COR_6$ est un radical acide, ester ou amide, suivie de la déprotection du radical carboxyle;

d) lorsqu'il est requis de préparer un composé de formule (I) où $R_1$ est CH=CH, la réaction d'un composé de formule (V) tel que défini ci-dessus avec l'acide malonique ou un ester de l'acide malonique;

e) l'hydrolyse d'un composé de formule (VI):

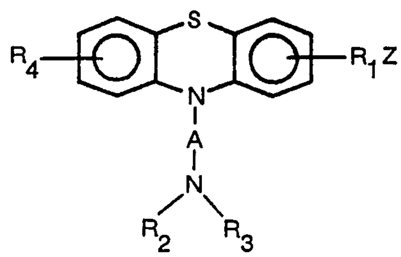

(VI)

où $R_2$ à $R_4$ et A sont tels que définis ci-dessus et $R_1Z$ est un radical $R_1CN$ où $R_1$ est un radical hydrocarboné bivalent en $C_{1-7}$ ou un radical $R_7$—CH=C(Y)XH où $R_7$ est un radical hydrocarboné bivalent en $C_{1-6}$ ou une liaison simple, X est oxygène ou soufre et Y est un radical amino;

f) l'alcoylation d'un composé de formule (VII):

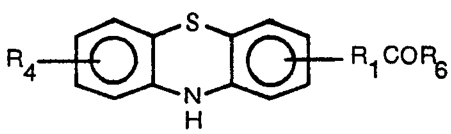

(VII)

où $R_1$ et $R_4$ sont tels que définis ci-dessus et $COR_6$ est un radical ester ou amide, suivie de la déprotection; et ensuite, la conversion éventuelle d'un composé de formule (I) en un autre composé de formule (I) suivant des procédés connus de l'homme de métier, par exemple la réduction d'une ou de plusieurs doubles liaisons.

2. Procédé suivant la revendication 1 pour préparer un composé de formule (II)

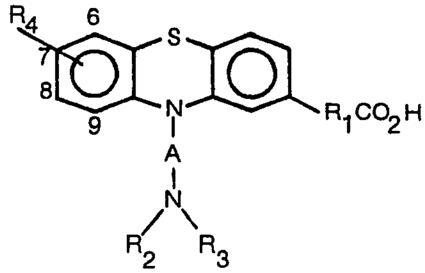

(II)

ou un sel de celui-ci,

où $R_1$ et $R_4$ et A sont tels que définis pour la formule (I).

3. Procédé suivant la revendication 1 pour préparer un composé suivant la revendication 1 ou 2, dans lequel $R_1$ est un radical $-(CH_2)_n-$ où n est un entier de 0 à 3, ou un radical —CH=CH, ou un radical $-CH(CH_3)(CH_2)_m-$ où m est 0 ou 1, un radical $-C(CH_3)_2-$.

4. Procédé suivant la revendication 1 pour préparer un composé suivant l'une quelconque des revendications 1 à 3, dans lequel —$NR_2R_3$ est un radical diméthylamino ou diéthylamino.

5. Procédé suivant la revendication 1 pour préparer un composé suivant l'une quelconque des revendications 1 à 4, dans lequel $R_4$ est hydrogène, méthyle, chloro ou fluoro.

6. Procédé suivant la revendication 1 pour préparer un composé suivant l'une quelconque des revendications 1 à 5, dans lequel A est un radical éthylène ou n- ou iso-propylène.

7. Procédé suivant la revendication 1 pour préparer un composé de formule (I) choisi parmi:
l'acide trans-β-[10-2-(2-diméthylaminopropyl)phénothiazin-2-yl]acrylique
l'acide [10-(2-diméthylaminopropyl)phénothiazin-2-yl]acétique
l'acide [10-(2-diméthylaminopropyl)phénothiazin-2-yl]propionique
l'acide 10-(2-diméthylaminopropyl)phénothiazine-3-carboxylique
la *E*-3-(2-carboxyvinyl)-10-(2-diméthylaminopropyl)phénothiazine
la 3-(2-Carboxyéthyl)-10-(2-diméthylaminopropyl)phénothiazine
l'acide 10-(2-diméthylaminopropyl)phénothiazine-4(ou 1)-carboxylique
la (*E*)-4(ou 1)-(2-carboxyvinyl)-10-(2-diméthylaminopropyl)phénothiazine
la 4-(ou 1)-(2-carboxyéthyl)-10-(2-diméthylaminopropyl)phénothiazine
l'acide [7-chloro-10-(2-diméthylaminopropyl)-2-phénothiazinyl]acétique
l'acide [10-(2-diméthylaminoéthyl)-7-méthyl-2-phénothiazinyl]acétique
l'acide [10-(2-diméthylaminoéthyl)-7-fluoro-2-phénothiazinyl]acétique
l'acide 2-[10-(2-diméthylaminopropyl)-2-phénothiazinyl]-2-méthylpropionique
l'acide 2-[10-(3-diméthylaminopropyl)-2-phénothiazinyl]-2-méthylpropionique
l'acide 2-méthyl-2-[10-[3-(N-pyrrolidino)propyl]-2-phénothiazinyl]propionique
l'acide 10-(2-diméthylaminopropyl)phénothiazine-2-carboxylique
et leurs sels.

8. Procédé pour préparer une composition pharmaceutique qui comprend la mise en mélange d'un composé de formule (I) suivant la revendication 1 avec un ou plusieurs excipients pharmaceutiquement acceptables.

9. Procédé suivant la revendication 8, dans lequel d'autres constituants thérapeutiques sont mis en mélange avec le composé de formule (I) et les excipients pharmaceutiquement acceptables.